# EUROPEAN PATENT SPECIFICATION

(11) **EP 2 453 743 B1**
(45) Date of publication and mention of the grant of the patent: **12.04.2017**
(21) Application number: 10800558.8
(22) Date of filing: 15.07.2010
(51) Int. Cl.: A01N 37/12, A01N 37/44, A61K 31/198, A61K 31/167, A61K 9/00, A61K 9/20

(54) **N-ACETYL CYSTEINE COMPOSITIONS AND THEIR USE IN IMPROVING THE THERAPEUTIC EFFICACY OF ACETAMINOPHEN**
N-ACETYL-CYSTEINZUSAMMENSETZUNGEN UND IHRE VERWENDUNG ZUR VERBESSERUNG DER THERAPEUTISCHEN WIRKUNG VON ACETAMINOPHEN
COMPOSITIONS DE N-ACÉTYL CYSTÉINE ET LEUR UTILISATION POUR AMÉLIORER L'EFFICACITÉ THÉRAPEUTIQUE DE L'ACÉTAMINOPHÈNE

(30) Priority: 15.07.2009 US 225679 P
(43) Date of publication of application: 23.05.2012
(73) Proprietor: The Board of Trustees of The Leland Stanford Junior University, Palo Alto, CA 94306-11065 (US)
(72) Inventor: HERZENBERG, Leonore, A., Stanford CA 94305 (US); ANDRUS, James, Redwood City CA 94061 (US)
(74) Representative: Beresford, Keith Denis Lewis
(86) International application number: PCT/US2010/042163
(87) International publication number: WO 2011/008976

(56) References cited:
- US-A1- 2002 142 991
- US-A1- 2008 139 654
- US-A1- 2008 139 654
- US-A1- 2008 226 715
- "Acetaminophen in aSAH to Inhibit Lipid Peroxidation and Cerebral Vasospasm", , 26 December 2007 (2007-12-26), pages 1-4, XP55043637, Retrieved from the Internet: URL:http://www.clinicaltrials.gov/ct2/show /record/NCT00585559?term=paracetamol+and+a cetyl+cysteine&rank=2 [retrieved on 2012-11-09]
- DATABASE BIOSIS [Online] BIOSCIENCES INFORMATION SERVICE, PHILADELPHIA, PA, US; 1992, IVERSEN H K: "N ACETYLCYSTEINE ENHANCES NITROGLYCERIN-INDUCED HEADACHE AND CRANIAL ARTERIAL RESPONSES", XP002686850, Database accession no. PREV199294126913 & IVERSEN H K: "N ACETYLCYSTEINE ENHANCES NITROGLYCERIN-INDUCED HEADACHE AND CRANIAL ARTERIAL RESPONSES", CLINICAL PHARMACOLOGY AND THERAPEUTICS, vol. 52, no. 2, 1992, pages 125-133, ISSN: 0009-9236
- SA'ED H. ZYOUD ET AL: "N-acetylcysteine-induced headache in hospitalized patients with acute acetaminophen overdose", FUNDAMENTAL & CLINICAL PHARMACOLOGY, vol. 25, no. 3, 1 June 2011 (2011-06-01), pages 405-410, XP55043698, ISSN: 0767-3981, DOI: 10.1111/j.1472-8206.2010.00831.x
- "Physicians' desk reference", 1 January 2005 (2005-01-01)
- "Levine's pharmacology drug actions and reactions", pages 213-213,

## Description

### CROSS REFERENCE TO RELATED APPLICATIONS

This application claims the benefit of U.S. Provisional Application No. 61/225,679, filed July 15. 2009.

### FIELD OF THE INVENTION

The described invention relates to compositions for improving the efficacy of acetaminophen formulations comprising a therapeutic efficacy-improving amount of N-acetylcysteine and a unit dose of acetaminophen, and use of such compositions for analgesic and antipyretic applications, whereby the formulations of the compositions enable increased therapeutic efficacy of acetaminophen when administered at standard doses and enable administration of lower than standard doses of acetaminophen to achieve efficacy equivalent to that obtained at a standard acetaminophen dose.

### BACKGROUND OF THE INVENTION

Over the past thirty years, acetaminophen, in its various formulations, has become the largest selling over the counter (OTC) drug throughout the world. It presently constitutes 35% of the analgesic market in North America, excluding other preparations that contain acetaminophen. During that same time, acetaminophen overdose also has become the most common drug intoxication and is responsible for more emergency room visits than any other medicine. It also is the leading cause of hepatic failure in the United States; accounting for 39% of all cases in a review of tertiary care centers. This presents a strain on the medical system for specialized medical services and has significant financial ramifications.

Acetaminophen initially was not thought to present toxicity risks when used at recommended doses. However, more recent studies demonstrate significant toxicity at recommended doses, both in healthy individuals and in persons with diseases or other conditions that result in glutathione (GSH) depletion. Such GSH deficiency has been reported in many disease conditions. Persons with GSH deficiency potentially arc at greater risk of Acetaminophen toxicity at doses of acetaminophen within recommended dosing levels.

### Acetaminophen toxicity and glutathione deficiency.

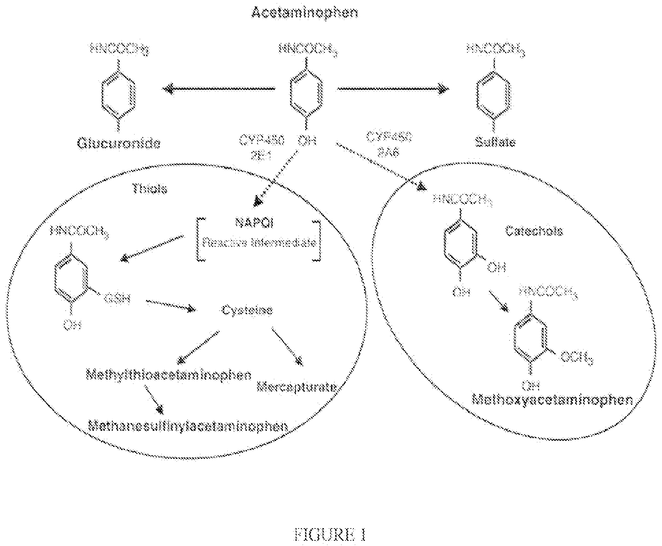

N-acetyl-para-aminophenol ("APAP'', "acetaminophen" or "paracetamol") is absorbed rapidly in the stomach and duodenum and reaches its peak levels approximately 1 to 2 hours after ingestion. It has a volume of distribution of 0.9-1.0 L/kg and binds to erythrocytes and only slightly to protein. APAP degradation occurs in the liver by several mechanisms. APAP can undergo glucuronidation or sulfation to form non-toxic hydrophilic metabolites that are renally excreted, while the CYP2E1, CYP1A2, and CYP3A4 subfamilies of the cytochrome P-450 pathway metabolize the remainder of the APAP to N-acetyl-p-benzoquinone-imine ("NAPQI"). NAPQI then conjugates with reduced GSH to form non-toxic mercapturic acid, which also is renally excreted. (Figure 1). The degree to which each pathway is utilized is dependent on several factors, including the age of the individual and the presence and degree of hepatic dysfunction.

Acetaminophen toxicity develops once the three detoxifying pathways have become overloaded and the NAPQI present exceeds the reduced GSH stores. In acetaminophen overdose, GSH stores become consumed completely thus overwhelming the liver's ability to detoxify NAPQI. The remaining unconjugated NAPQI then binds to the proteins of hepatocytes, which leads to centrilobular hepatic necrosis. In addition, GSH depletion renders hepatocytes (and other cells) more sensitive to attack by cytokines, which can be produced in the liver and elsewhere pursuant to events that follow overrun of the ability to detoxify NAPQI.

The liver requires GSH in its reduced form for complete, non-toxic clearance of acetaminophen. Reduced GSH constitutes the majority of the intracellular glutathione in the body. Oxidized GSH (GSSG) is formed during normal oxidative metabolism. It also is produced when cells are subjected to oxidative stress or to exogenous toxins that arc detoxified by conjugation to GSH. GSSG and GSH conjugates are released rapidly from cells and excreted from the body: relatively little GSSG is recycled to GSH. GSH levels commonly arc measured by HPLC or mass spectrometry as total GSH reduced and extracted from circulating erythrocytes (or separately as erythrocyte GSH and GSSG).

Replenishment of reduced GSH requires an exogenous thiol supply, which usually is acquired by ingestion of cysteine, cystine or methionine in protein or other form. It also can be acquired by ingestion of NAC, which is used to treat GSH deficiency caused by acetaminophen overdose, because it is rapidly converted to cysteine during first pass metabolism in the liver.

Stores of reduced GSH are influenced greatly by nutritional status, presence of certain disease states, and exposures to oxidative stressors and molecules that are detoxified by conjugation with GSH. Viral, bacterial, and fungal infections, malnutrition, chronic and acute alcohol consumption, diabetes, certain metabolic diseases, and consumption of oxidative drugs all have been shown to decrease GSH. Table 1 is a noninclusive list of diseases in which GSH deficiency has been demonstrated.

**Table 1. Diseases in which GSH Deficiency Has Been Demonstrated**

| **Classification** | **Disease** |
|---|---|
| Hepatic Function | Acetaminophen toxicity |
| | Alcohol |
| | Hepatitis |
| Renal Function | Chronic Kidney Failure |
| | Dialysis |
| | Nephrotoxicity |
| | Alpha-Amanintin |
| Cardiovascular | Angina |
| | Arteriosclerosis/Cardiac Risk |
| | Myocardial Infarction |
| | Cardiomyopathy |
| Endocrine | Diabetes |
| Pulmonary | Bronchopulmonary |
| | ARDS |
| | Fibrosing Alveolitis |
| | Chronic Asthma |
| | Chronic Bronchitis/COPD |
| | Cystic Fibrosis |
| | Pulmonary Fibrosis |
| | Smoking |
| | Lung Cancer |
| Critical Care | Intensive Care |
| | Sepsis/Septic Shock |
| | Malnutrition |
| Infection | HIV |
| | Flelicobacter pylori |
| | influenza |
| | Malaria |
| | Epilepsy |
| Gastrointestinal | Inflammatory Bowel Disease |
| | Barrett's Esophagus |
| | Liver Disease |
| | Liver Transplantation |
| | Colon Cancer |
| Optic | Blepharitis |
| | Cataract |
| | Eale's Disease |
| Skin | Psoriasis |
| | Photodermatitis |
| Immune system | Rheumatoid Arthritis |
| | Comm. Variable Immunodeficiency |
| Urogenital | Prostate |
| | Urinary |
| Muscular | Exercise |
| Aging | |
| Toxic Agents | Arsenic Poisoning |
| | Other Chemicals and Medications |
| Perinatal | Preeclampsia |
| | Neonate |
| Metabolism | Phenylketonuria |

The disease states in which GSH-deficiency has been documented parallel the disease states in which acetaminophen toxicity has been documented at or below recommended dose levels. Therefore disease states in which GSH deficiency has been documented are likely to place the body at greater risk for acetaminophen toxicity and, conversely, depletion of GSH due to acetaminophen consumption may predispose to, or exacerbate, these disease states.

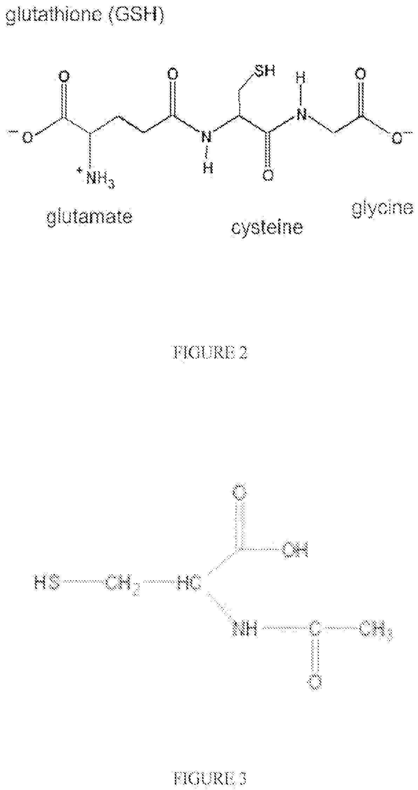

Acetaminophen toxicity is treated by rapid administration of N-acetylcysteine (NAC), a cysteine prodrug that supplies the cysteine necessary for the synthesis of glutathione (GSH), a key intracellular tri-peptide that is consumed by removal of the highly toxic acetaminophen metabolite, N-acetyl-p-benzoquinone-imine (NAPQI). (Figures 2 and 3). GSH is essential to cell survival. It plays key roles in cellular metabolism and protection against oxidative and other toxic molecules, including those generated in response to attack by cytokines that induce pain and fever. Administered rapidly enough, NAC completely restores GSH leaving no residual toxicity. Beneficial effects of NAC treatment for acetaminophen overdose strongly imply GSH depletion as the key component of acetaminophen toxicity, since GSH repletion is by far the most important effect of NAC treatment.

It is known that decreased levels of GSH are associated with increased pain and fever while increased levels of GSH are associated with decreased pain and fever. Consistent with this inverse relationship between GSH levels and signs of inflammation (pain and/or fever), decreasing GSH renders cells more sensitive to the effects of IL-1, IL-6 and TNF, all of which participate in increasing pain and fever. Thus, although acetaminophen acts directly to decrease pain and fever, its detoxification, which depletes GSH, tends to negate the beneficial effect of the drug. It also is known that enteral NAC decreases levels of IL-1, IL-6 and TNF by restoring GSH. NAC, which restores GSH, has been shown to reduce fever and pain.

Since GSH depletion becomes more pronounced as larger doses of acetaminophen are consumed, acetaminophen efficacy can be expected to decrease progressively with increasing doses of the drug. The dose at which efficacy is significantly impaired will differ, depending on patient GSH levels at the time the drug is administered. However, in all cases, increased acetaminophen dosing is likely to yield diminishing returns vis a vis fever and pain control.

U.S. Pat. No. 6,566,401 discloses that combined formulation of acetaminophen at a standard unit dose with NAC compared to APAP alone decreases the toxicity of inadvertent or intentional overdose and prevents toxicity associated with acetaminophen dosing within acceptable ranges. Whether NAC interferes with APAP. efficacy has not been well studied.

The problems of hepatic dysfunction and acute hepatic failure due to APAP toxicity have raised significant concerns with physicians, the Food and Drug Administration, and the general public regarding the safety of this highly popular over-the-counter (OTC) drug, which has led the Food and Drug Administration to require explicit overdose warnings, including the risk of hepatotoxicity to those who consume three or more alcoholic beverages per day. The safety of acetaminophen in nursing mothers or in pregnancy is not known.

The Physician's Desk Reference Primary Care Resource Center (www.pdr.net, visited 7/11/09) listing for acetaminophen sold as the brand TYLENOL® show the following unit doses and maximum daily doses obtained from the FDA monograph for acetaminophen:

| **Age** | **Body Weight** | **Mg per Dose** | **Maximum Daily Dose** |
|---|---|---|---|
| 0-3 months | 2.72-4.99 kg (6-11 lb) | 40 mg | 200 |
| 4-11 months | 5.44-7.71 kg (12-17 lb) | 80 mg | 400 |
| 12-23 months | 8.16-10.43 kg (18-23 lb) | 120 mg | 600 |
| 2-3 years | 10.89-15.88 kg (24-35 lb) | 160 mg | 800 |
| 4-5 years | 16.33-21.32 kg (36-47 lb) | 240 mg | 1200 |
| 6-8 years | 21.77-26.76 kg (48-59 lb) | 320 mg | 1600 |
| 9-10 years | 27.22-32.21 kg (60-71 lb) | 400 mg | 2000 |
| 11 years | 32.66-43.09 kg (72-95 lb) | 480 mg | 2400 |
| 12 years | 43.55 kg (96 lb) | 640 mg | 3200 |
| 6-11 years | | 325 mg | 1625 (Regular Strength) |
| >12 to adult | | 650 mg | 3900 (Regular Strength only) |
| | | | 4000 (Arthritis Pain and 8 Hour) |
| >12 to adult | | 500 mg | 4000 |

Acetaminophen is available over the counter in several dose forms, including drops (80 mg in 0.8 ml), Children's Suspension (160 mg/5 ml); Extra-Strength solution (500 mg/15 ml); chewable (80 mg and 160 mg); tablet, Regular Strength (325 mg); Extra Strength Easy Tabs, GoTabs, caplets, Cool Caplets, and rapid release gels (500 mg); and caplets and gel tabs (650 mg).

The most common causes of unintentional acetaminophen overdose are (1) Subjects taking several products, unaware that each one contains the maximum safe dose of APAP; (2) Subjects abusing narcotics often do not realize that the drug is paired with APAP. As their tolerance for the narcotic increases, they take larger doses, and get huge doses of APAP in the process. (3) Subjects in pain take more and more pain reliever to get relief, far exceeding the recommended dose. Because APAP is sold over the counter, users and those administering acetaminophen to children and elderly adults falsely believe that swallowing a few more pills than the recommended dose is safe. (4) Chronic alcoholics and chronically ill patients are more sensitive to APAP toxicity. The threat of accidental acetaminophen overdose is especially high amongst the elderly population.

Dosing miscalculations are a common occurrence with respect to both a child's age and weight associated with the purchase of incorrect over the counter medications. Hospitals have reported numerous instances where inadvertent overdosing was due to the use of adult APAP formulations (which are on average three times as potent as children's formulations) instead of children's formulations. Such dosing miscalculations arc particularly common when there are children of multiple age levels living in the same household.

While the hazardous combination of APAP and alcohol generally is known amongst chronic alcoholics, occasional drinkers arc ignorant of the danger. It is commonplace to reach for bottle of pain relievers to alleviate hangover symptoms such as headache, nausea and dizziness, but doing so can cause liver damage and even death. The liver uses the same biochemical pathway to remove APAP and alcohol, which, when APAP and alcohol arc combined, becomes saturated, rendering liver tissue defenseless.

Since 1977, on numerous occasions, the FDA has commissioned the Non-Prescription Drug Advisory Committee to review evidence on acute liver failure due to acetaminophen overdose, and to propose recommendations to increase patient safety when consuming APAP. A number of label changes have resulted. Following the committee meeting in 2002, the Committee unanimously agreed that FDA action is necessary to protect consumers from the dangers of APAP. In light of the Committee's findings; the FDA launched a campaign at the pharmacy level in January 2004 to raise public awareness on the dangers of APAP toxicity.

More recently, a federal advisory panel has recommended that the FDA take action to decrease illness and death due to APAP. toxicity by banning well-used APAP-narcotic preparations and by decreasing the maximal allowable daily and single APAP dose. The panel recommendation, with which Tylenol® producer Johnson & Johnson "strongly disagrees" is based on an annual reported mortality and morbidity rates in the U.S. of 400 deaths per year and 42,000 hospitalizations per year due to inadvertent and intentional APAP overdoses. The panel recommendations mark a clear change in philosophy regarding the use of APAP.

Physicians, acetaminophen manufacturers and the public are concerned that decreasing the allowable acetaminophen dose will withdraw important pain and fever relief from patients in need of such. Physicians treating high fevers in children, for example, often find their treatment options limited by the currently allowed dosage. Decreasing this will further limit treatment in these cases. Similarly, treatment of severe pain may be limited by the maximum dosage of acetaminophen that can be prescribed or recommended without causing toxicity.

The described invention addresses this problem. It describes compositions that improve the therapeutic efficacy of a given dose of acetaminophen comprising acetaminophen and N-acetylcysteine, whereby the maximum daily dose of acetaminophen in the described compositions is less than the maximum daily dose of acetaminophen alone, but the compositions therapeutically are as effective as compositions that contain the maximum daily dose of acetaminophen alone. Benefits that can be realized by the proposed compositions include, but are not limited to, decreasing the amount of acetaminophen necessary to achieve a given clinical end and increasing the efficacy of an acetaminophen dose, including, but not limited to, the maximum allowable dose.

### BRIEF DESCRIPTION OF THE DRAWINGS

Figure 1 shows the pathways for metabolism of acetaminophen.
Figure 2 shows the structural formula for glutathione.
Figure 3 shows the structural formula for NAPQI.
Figure 4 shows temporal characteristics of a drug effect and its relationship to the therapeutic window. *a* = onset of effect; *b* = peak effect; *c* = intensity; *d* = MEC for adverse response; *e* = therapeutic window; *f*= MEC for desired response; and g = duration of action.

### SUMMARY

According to a first aspect, the invention provides a composition for use in a method of treating pain in a subject in need of analgesic relief over a course of therapy, as defined in claim 1. According to a second aspect, the invention provides a composition for use in a method of treating fever in a subject in need of antipyretic relief over a course of therapy, as defined in claim 2. According to a third aspect, the invention provides a composition for use in a method for improving therapeutic efficacy of acetaminophen at a less than standard maximum daily dose as defined in claim 7.

According to an embodiment, the composition is an oral formulation selected from a liquid solution, a syrup, an elixir, a suspension, an emulsion, a tablet, a capsule, a sustained release formulation, or a powder. According to another embodiment, the tablet is a compressed tablet. According to another embodiment, the tablet is a coated tablet. According to another embodiment, the tablet is an effervescent tablet.

### DETAILED DESCRIPTION OF THE INVENTION

The described invention provides compositions for improving the therapeutic efficacy of acetaminophen comprising (a) a unit dose of acetaminophen and (b) N-acetylcysteine, pharmaceutical salts of N-acetylcysteine, and derivatives of N-acetylcysteine (hereinafter collectively referred to as "N-acetylcysteine"), wherein the ratio of acetaminophen to N-acetyl cysteine in the compositions ranges from about 1:15 to about 1:0.000977; and methods of use thereof. The therapeutic efficacy of the described acetaminophen-NAC compositions does not decrease during long term use of the acetaminophen-NAC composition.

The term "administering", meaning the giving or supplying of a medication, as used herein includes *in vivo* administration, as well as administration directly to tissue *ex vivo.* Generally, compositions may be administered systemically either orally, buccally, parenterally, topically, by inhalation or insufflation (i.e., through the mouth or through the nose), or rectally in dosage unit formulations containing conventional nontoxic pharmaceutically acceptable carriers, adjuvants, and vehicles as desired, or maybe locally administered by means such as, but not limited to, injection, implantation, grafting, topical application, or parenterally.

The term "derivative" refers to a compound that arises from a parent compound by replacement of an atom with another atom or group of atoms. A derivative of N-acetylcysteine has the same acetaminophen-efficacy improving activity as does N-acetylcysteirie.

The term "disease" or "disorder", as used herein, refers to an impairment of health or a condition of abnormal functioning. The term "syndrome," as used herein, refers to a pattern of symptoms indicative of some disease or condition. The term "injury," as used herein, refers to damage or harm to a structure or function of the body caused by an outside agent or force, which may be physical or chemical. The term "condition", as used herein, refers to a variety of health states and is meant to include disorders or diseases caused by any underlying mechanism or disorder, injury, and the promotion of healthy tissues and organs.

The terms "dose" and "dosage" are used interchangeably and mean the quantity of a drug or other remedy to be taken or applied all at one time or in fractional amounts within a given period.

The term "drug" as used herein refers to a therapeutic agent or any substance, other than food, used in the prevention, diagnosis, alleviation, treatment, or cure of disease.

As used herein, the terms "formulation" and "composition" are used interchangeably. The terms "drug" and "pharmaceutical" also are used interchangeably to refer to a pharmacologically active substance or composition. These terms of art arc well-known in the pharmaceutical and medicinal arts.

As used herein the term "inflammation" refers to a physiologic response to infection and injury in which cells involved in detoxification and repair are mobilized to the compromised site by inflammatory mediators. The classic signs of inflammation are pain (dolor), heat (calor), redness (rubor), swelling (tumor), and loss of function (functio laesa). Histologically, inflammation involves a complex series of events, including dilatation of arterioles, capillaries, and venules, with increased permeability and blood flow; exudation of fluids, including plasma proteins; and leukocytic migration into the inflammatory focus.

The term "acute inflammation" as used herein, refers to inflammation, usually of sudden onset, characterized by the classical signs, with predominance of the vascular and exudative processes. The term "Chronic inflammation" as used herein refers to inflammation of slow progress and marked chiefly by the formation of new connective tissue; it may be a continuation of an acute form or a prolonged low-grade form, and usually causes permanent tissue damage.

Regardless of the initiating agent, the physiologic changes accompanying acute inflammation encompass four main features: (1) vasodilation, which results in a net increase in blood flow, is one of the earliest physical responses to acute tissue injury; (2) in response to inflammatory stimuti, endothelial cells lining the venules contract, widening the intracellular junctions to produce gaps, leading to increased vascular permeability which permits leakage of plasma proteins and blood cells out of blood vessels; (3) inflammation often is characterized by a strong infiltration of leukocytes at the site of inflammation, particularly neutrophils (polymorphonuclear cells). These cells promote tissue damage by releasing toxic substances at the vascular wall or in uninjured tissue; and (4) fever, produced by pyrogens released from leukocytes in response to specific stimuli.

During the inflammatory process, soluble inflammatory mediators of the inflammatory response work together with cellular components in a systemic fashion in the attempt to contain and eliminate the agents causing physical distress. The term "inflammatory mediators." as used herein refers to the molecular mediators of the inflammatory process. These soluble, diffusible molecules act both locally at the site of tissue damage and infection and at more distant sites. Some inflammatory mediators are activated by the inflammatory process, while others arc synthesized and/or released from cellular sources in response to acute inflammation or by other soluble inflammatory mediators. Examples of inflammatory mediators of the inflammatory response include, but arc not limited to, plasma proteases, complement, kinins, clotting and fibrinolytic proteins, lipid mediators, prostaglandins, Icukotrienes, platelet-activating factor (PAF), peptides and amines, including, but not limited to, histamine, serotonin, and neuropeptides, proinflammatory cytokines, including, but not limited to, interleukin-1, interleukin-4, interleukin-6, interleukin-8, tumor necrosis factor (TNF), interferon-gamma, and interleukin 12.

The term "modulate" as used herein means to regulate, alter, adapt, or adjust to a certain measure or proportion.

The terms "subject" or "individual" or "patient" are used interchangeably to refer to a member of an animal species of mammalian origin that may benefit from the administration of a drug composition or method of the described invention. Examples of subjects include humans, and other animals such as horses, pigs, cattle, dogs, cats, rabbits, and aquatic mammals.

"Subjects in need thereof" are subjects ordinarily treated with acetaminophen alone for anti-pyresis or analgesia that may benefit from the described invention and include (but are not limited to): subjects buffering from acute hepatic failure of unknown etiology; subjects suffering from a disease or condition associated with GSH deficiency; subjects whose serum levels of acetaminophen indicate non-toxic levels but who are at increased risk for APAP toxicity due to oxidative stress or other aspects of the patient's condition; patients having certain viral infections; patients having taken a toxicity producing amount of a drug that causes oxidative stress or subject related decreases in cysteine-glutathione levels, such as oxidizing antibiotics. e.g., sulfa, chloramphenicol, erythromycin, and antiviral agent, and patients under oxidative stress or that otherwise have severe illness that makes them more susceptible than a normal subject would be to acetaminophen toxicity, including, for example, but not limited to, patients with septic shock, distributive shock, hemorrhagic shock, acute respiratory distress syndrome, organ failure, and closed head injury.

The term "therapeutically effective amount" or an "amount effective" of one or more active agent(s) is an amount that is sufficient to provide the intended benefit of treatment. Dosage levels are based on a variety of factors, including the type of injury, the age, weight, sex, medical condition of the patient, the severity of the condition, the route of administration, and the particular active agent employed. Thus the dosage regimen may vary widely, but can be determined routinely by a physician using standard methods.

The term "therapeutic efficacy enhancing amount" refers to the amount necessary or sufficient to realize the desired biologic effect of enhanced therapeutic efficacy.

The term "therapeutic agent" as used herein refers to a drug, molecule, composition or other substance that provides a therapeutic effect. The term "active" as used herein refers to the ingredient, component or constituent of the compositions of the present invention responsible for the intended therapeutic effect. The terms "therapeutic agent" and "active agent" are used interchangeably herein.

The term "therapeutic component" as used herein refers to a therapeutically effective dosage (i.e., dose and frequency of administration) that eliminates, reduces, or prevents the progression of a particular disease manifestation in a percentage of a population. An example of a commonly used therapeutic component is the ED₅₀, which describes the dose in a particular dosage that is therapeutically effective for a particular disease manifestation in 50% of a population.

The term "therapeutic effect" as used herein refers to a consequence of treatment, the results of which are judged to be desirable and beneficial. A therapeutic effect may include, directly or indirectly, the arrest, reduction, or elimination of a disease manifestation. A therapeutic effect also may include, directly or indirectly, the arrest reduction or elimination of the progression of a disease manifestation.

The terms "treat" or "treating" as used herein refer to accomplishing one or more of the following: (a) reducing the severity of the disorder; (b) limiting development of symptoms characteristic of the disorder(s) being treated; (c) limiting worsening of symptoms characteristic of the disorder(s) being treated; (d) limiting recurrence of the disorder(s) in patients that have previously had the disorder(s); and (e) limiting recurrence of symptoms in patients that were previously asymptomatic for the disorder(s).

According to a first aspect, the invention provides a composition for use in a method of treating pain in a subject in need of analgesic relief over a course of therapy, as defined in claim 1. According to a second aspect, the invention provides a composition for use in a method of treating fever in a subject in need of antipyretic relief over a course of therapy, as defined in claim 2.

According to one embodiment of the composition, the unit dose of acetaminophen in (a) is such that a total of all unit doses administered per day is an amount less than a standard maximum daily dose of acetaminophen, wherein the standard maximum daily dose of acetaminophen is: 200 mg for a subject 0-3 months of age, 2.72-4.99 kg body weight, receiving 40 mg/dose; 400 mg for a subject 4-11 months of age, 5.44-7.71 kg body weight, receiving 80 mg/dose; 600 mg for a subject 12-23 months of age, 8.16-10.43 kg body weight, receiving 120 mg/dose; 800 mg for a subject 2-3 years of age, 10.89-15.85 kg body weight, receiving 160 mg/dose; 1200 mg for a subject 4-5 years of age, 16.33-21.32 kg body weight, receiving 240 mg/dose; 1600 mg for a subject 6-8 years of age, 21.77-26.76 kg body weight, receiving 320 mg/dose; 1625 mg for a subject 6-11 years of age, receiving 325 mg/dose; 2000 mg for a subject 9-10 years of age, 27.22-32.21 kg body weight, receiving 400 mg/dose; 2400 mg for a subject 11 years of age, 32.66-43.09 kg body weight, receiving 480 mg/dose; 3200 for a subject 12 years of age, 43.55 kg, body weight receiving 640 mg/dose; 4000 mg for a subject >12 years of age to adult, receiving 650 mg/dose; 4000 mg for a subject >12 years of age to adult, receiving 500 mg/dose;and the therapeutic effectiveness of the composition is equivalent to the therapeutic effectiveness of acetaminophen alone when acetaminophen is administered at the standard maximum daily dose of acetaminophen.

As used herein, the term "efficacy-enhancing" means improving the therapeutic effectiveness of a therapeutic agent.

Molar ratios of acetaminophen and N-acetylcysteine are determined using the molecular weight of APAP as 151, and the molecular weight of NAC as 163.

According to one embodiment, the molar ratio of APAP:NAC in the composition of the invention is 1:15 (w/w). According to another embodiment, the molar ratio of APAP:NAC in the composition is 1:10 (w/w). According to another embodiment, the molar ratio of APAP:NAC in the composition is 1:5 (w/w). According to another embodiment, the molar ratio of APAP:NAC in the composition is 1:4 (w/w). According to another embodiment, the molar ratio of APAP:NAC in the composition is 1:3 (w/w). According to another embodiment, the molar ratio of APAP:NAC in the composition is 1:2 (w/w). According to another embodiment, the molar ratio of APAP:NAC in the composition is 1:1 (w/w) According to another embodiment, the molar ratio of APAP:NAC in the composition is 1:0.5 (w/w). According to another embodiment, the molar ratio of APAP:NAC in the composition is 1:0.25 (w/w). According to another embodiment, the molar ratio of APAP:NAC in the composition is 1:0.125 (w/w). According to another embodiment, the molar ratio of APAP:NAC in the composition is 1: 0.625 (w/w). According to another embodiment, the molar ratio of APAP:NAC in the composition is 1: 0.03125 (w/w). According to another embodiment, the molar ratio of APAP:NAC in the composition is 1:0.015625 (w/w). According to another embodiment, the molar ratio of APAP:NAC in the composition is 1:0.0078125 (w/w). According to another embodiment, the molar ratio of APAP:NAC in the composition is 1:0.00390625 (w/w). According to another embodiment, the molar ratio of APAP:NAC in the composition is 1:0.001953 (w/w). According to another embodiment, the molar ratio of APAP:NAC in the composition is 1:0.000977 (w/w).

Nonlimiting examples of some embodiments of a unit dose of a composition according to the described invention comprising 40 mg APAP and an acetaminophen therapeutic efficacy-enhancing amount of NAC are shown in Table 2.

**Table 2. APAP-NAC compositions comprising 40 mg APAP**

| Molar ratio APAP:NAC | Mg APAP | mg NAC |
|---|---|---|
| 1:1 | 40 | 40.75 |
| 1:0.5 | 40 | 20.4 |
| 1:0.25 | 40 | 10.3 |
| 1:0.125 | 40 | 5.15 |

Nonlimiting examples of some embodiments of unit dose of a composition according to the described invention comprising 80 mg APAP and an acetaminophen therapeutic efficacy-enhancing amount of NAC arc shown in Table 3.

**Table 3. APAP-NAC compositions comprising 80 mg APAP**

| Molar ratio APAP:NAC | Mg APAP | mg NAC |
|---|---|---|
| 1:1 | 80 | 81.5 |
| 1:0.5 | 80 | 40.75 |
| 1:0.5 | 80 | 20.4 |
| 1:0.125 | 80 | 10.3 |

Nonlimiting examples of some embodiments of a unit dose of a composition according to the described invention comprising 325 mg APAP and an acetaminophen therapeutic efficacy-enhancing amount of NAC are shown in Table 4.

**Table 4. APAP-NAC compositions comprising 325 mg APAP**

| Molar ratio APAP:NAC | Mg APAP | mg NAC |
|---|---|---|
| 1:1 | 325 | 358.6 |
| 1:0.5 | 325 | 179.8 |
| 1:0.25 | 325 | 97.8 |
| 1:0.125 | 325 | 48.9 |

Nonlimiting examples of some embodiments of a unit dose of a composition according to the described invention comprising 500 mg APAP and an acetaminophen therapeutic efficacy-enhancing amount of NAC arc shown in Table 5.

**Table 5. APAP-NAC compositions comprising 500 mg APAP**

| Molar ratio APAP:NAC | Mg APAP | mg NAC |
|---|---|---|
| 1:1 | 500 | 537.9 |
| 1:0.5 | 500 | 277.1 |
| 1:0.25 | 500 | 138.6 |
| 1:0.125 | 500 | 70.1 |

Nonlimiting examples of some embodiments of a unit dose of a composition according to the described invention comprising 650 mg APAP and an acetaminophen therapeutic efficacy-enhancing amount of NAC are shown in Table 6.

**Table 6. APAP-NAC compositions comprising 650 mg APAP**

| Molar ratio APAP:NAC | Mg APAP | mg NAC |
|---|---|---|
| 1:1 | 650 | 700.9 |
| 1:0.5 | 650 | 350.5 |
| 1:0.25 | 650 | 179.3 |
| 1:0.125 | 650 | 89.7 |

A skilled artisan can perform similar calculations for additional molar ratios.

Because NAC improves the therapeutic efficacy of APAP, the maximum daily dose of APAP in an APAP: NAC formulation may be reduced from the standard maximum daily dose of acetaminophen, wherein the standard maximum daily dose of acetaminophen is 200 mg/day, 400 mg/day, 600 mg/day, 800 mg/day, 1200 mg/day, 1600 mg/day, 1625 mg/day, 2000 mg/day, 2400 mg/day, 3200 mg/day, or 4000 mg/day, while having: the same efficacy as acetaminophen when administered at the standard maximum daily dose, without incurring the risk of toxicity due to oxidative stress or subject related decreases in cysteine/glutathione levels. According to one embodiment, the maximum allowable daily dose of APAP in a composition comprising APAP and NAC is less than 4000 mg because the composition has the same therapeutic effectiveness as when APAP is administered at a standard maximum allowable dose of 4000 mg. According to another embodiment, the maximum allowable daily dose of APAP in a composition comprising APAP and NAC is less than 3200 mg because the composition has the same therapeutic effectiveness as when APAP is administered at a standard maximum allowable dose of 3200 mg. According to another embodiment, the maximum allowable daily dose of APAP in a composition comprising APAP and NAC is less than 2400 mg because the composition has the same therapeutic effectiveness as when APAP is administered at a standard maximum allowable dose of 2400 mg. According to another embodiment, the maximum allowable daily dose of APAP in a composition comprising APAP and NAC is less than 2000 mg because the composition has the same therapeutic effectiveness as when APAP is administered at a standard maximum allowable dose of 2000 mg. According to another embodiment, the maximum allowable daily dose of APAP in a composition comprising APAP and NAC is less than 1600 mg because the composition has the same therapeutic effectiveness as when APAP is administered at a standard maximum allowable dose of 1600 mg. According to another embodiment, the maximum allowable daily dose of APAP in a composition comprising APAP and NAC is less than 1200 mg because the composition has the same therapeutic effectiveness as when APAP is administered at a standard maximum allowable dose of 1200 mg. According to another embodiment, the maximum allowable daily dose of APAP in a composition comprising APAP and NAC is less than 800 mg because the composition has the same therapeutic effectiveness as when APAP is administered at a standard maximum allowable dose of 800 mg. According to another embodiment, the maximum allowable daily dose of APAP in a composition comprising APAP and NAC is less than 600 mg because the composition has the same therapeutic effectiveness as when APAP is administered at a standard maximum allowable dose of 600 mg. According to another embodiment, the maximum allowable daily dose of APAP in a composition comprising APAP. and NAC is less than 400 mg because the composition has the same therapeutic effectiveness as when APAP. is administered at a standard maximum allowable dose of 400 mg. According to another embodiment, the maximum allowable daily dose of APAP in a composition comprising APAP and NAC is less than 200 mg because the composition has the same therapeutic effectiveness as when APAP is administered at a standard maximum allowable dose of 200 mg. According to another embodiment, the therapeutic efficacy of the described acetaminophen-NAC compositions does not decrease during long term use of the acetaminophen-NAC composition.

According to one embodiment, the increase in the therapeutic effectiveness of acetaminophen in an APAP:NAC composition is directly proportional to the therapeutic efficacy-enhancing amount of NAC. According to one embodiment, the increase in the therapeutic effectiveness of the acetaminophen is linearly proportional to the acetaminophen therapeutic efficacy-enhancing amount of NAG. According to another embodiment, the increase in the therapeutic effectiveness of the acetaminophen in an APAP:NAC composition is about 1.5-fold higher than that of the same acetaminophen dose administered alone. According to another embodiment, the increase in the therapeutic effectiveness of the acetaminophen in an APAP:NAC composition is about 2-fold higher than that of the same acetaminophen dose administered alone. According to another embodiment, the increase in the therapeutic effectiveness of the acetaminophen in an APAP:NAC composition is about 3-fold higher than that of the same acetaminophen dose administered alone. According to another embodiment, the increase in the therapeutic effectiveness of the acetaminophen in an APAP-NAC composition is about 4-fold higher than that of the same acetaminophen dose administered alone. According to another embodiment, the increase in the therapeutic effectiveness of the acetaminophen in an APAP:NAC composition is about 5-fold higher than that of the same acetaminophen dose administered alone. According to another embodiment, the increase in the therapeutic effectiveness of the acetaminophen in an APAP:NAC composition is about 6-fold higher than that of the same acetaminophen dose administered alone. According to another embodiment, the increase in the therapeutic effectiveness of the acetaminophen in an APAP:NAC composition is about 7-fold higher than that of the same acetaminophen dose administered alone. According to another embodiment, the increase in the therapeutic effectiveness of the acetaminophen in an APAP:NAC composition is about 8-fold higher than that of the same acetaminophen dose administered alone. According to another embodiment, the increase in the therapeutic effectiveness of the acetaminophen in an APAP:NAC composition is about 9-fold higher than that of the same acetaminophen dose administered alone. According to another embodiment, the increase in the therapeutic effectiveness of the acetaminophen in an APAP:NAC composition is about 10-fold higher than that of the same acetaminophen dose administered alone.

According to another embodiment, the therapeutic efficacy-enhancing amount of NAC is about 15-fold higher than the dose of acetaminophen. According to another embodiment, the therapeutic efficacy-enhancing amount of NAC is about 10-fold higher than the dose of acetaminophen. According to another embodiment, the therapeutic efficacy-enhancing amount of NAC is about 5-fold higher than the dose of acetaminophen According to another embodiment, the therapeutic efficacy-enhancing amount of NAC is about 2-fold higher than the dose of acetaminophen. According to another embodiment, the therapeutic efficacy-enhancing amount of NAC is about equal to the dose of acetaminophen. According to another embodiment, the therapeutic efficacy-enhancing amount of NAC is about one-fourth of the dose of acetaminophen. According to another embodiment, the therapeutic efficacy-enhancing amount of NAC is about one-eighth of the dose of acetaminophen. According to another embodiment, the therapeutic efficacy-enhancing amount of NAC is about one-sixteenth of the dose of acetaminophen. According to another embodiment, the therapeutic efficacy-enhancing amount of NAC is about one-thirty-second of the dose of acetaminophen. According to another embodiment, the therapeutic efficacy-enhancing amount of NAC is about one-sixty-fourth of the dose of acetaminophen.

According to another embodiment, the acetaminophen efficacy-enhancing amount of NAC is about 4000 mg/day. According to another embodiment, the acetaminophen efficacy-enhancing amount of NAC is about 3000 mg/day. According to another embodiment, the acetaminophen efficacy-enhancing amount of NAC is about 2000 mg/day. According to another embodiment, the acetaminophen efficacy-enhancing amount of NAC is about 1000 mg/day. According to another embodiment, the acetaminophen efficacy-enhancing amount of NAC is about 500 mg/day. According to another embodiment, the acetaminophen efficacy-enhancing amount of NAC is about 450 mg/day. According to another embodiment, the acetaminophen efficacy-enhancing amount of NAC is about 400 mg/day. According to another embodiment, the acetaminophen efficacy-enhancing amount of NAC is about 350 mg/day. According to another embodiment, the acetaminophen efficacy-enhancing amount of NAC is about 300 mg/day. According to another embodiment, the acetaminophen efficacy-enhancing amount of NAC is about 250 mg/day. According to another embodiment, the acetaminophen efficacy-enhancing amount of NAC is about 200 mg/day. According to another embodiment, the acetaminophen efficacy-enhancing amount of NAC is about 150 mg/day. According to another embodiment, the acetaminophen efficacy-enhancing amount of NAC is about 125 mg/day. According to another embodiment, the acetaminophen efficacy-enhancing amount of NAC is about 100 mg/day. According to another embodiment, the acetaminophen efficacy-enhancing amount of NAC is about 75 mg/day. According to another embodiment, the acetaminophen efficacy-enhancing amount of NAC is about 50 mg/day. According to another embodiment, the acetaminophen efficacy-enhancing amount of NAC is about 25 mg/day. According to another embodiment, the acetaminophen efficacy-enhancing amount of NAC is about 10 mg/day. According to another embodiment, the acetaminophen efficacy-enhancing amount of NAC is about 5 mg/day. According to another embodiment, the acetaminophen efficacy-enhancing amount of NAC is about 1 mg/day.

According to another embodiment, the molar ratio of the dose of acetaminophen to the therapeutic efficacy-enhancing amount of NAC present is 1:15. According to another embodiment, the molar ratio of the dose of acetaminophen to the therapeutic efficacy-enhancing amount of NAC present is 1:10. According to another embodiment, the molar ratio of the dose of acetaminophen to the therapeutic efficacy-enhancing amount of NAC present is 1:5. According to another embodiment, the molar ratio of the dose of acetaminophen to the therapeutic efficacy-enhancing amount of NAC present is 1:2. According to another embodiment, the molar ratio of the dose of acetaminophen to the therapeutic efficacy-enhancing amount of NAC present is 1:1. According to another embodiment, the molar ratio of the dose of acetaminophen to the therapeutic efficacy-enhancing amount of NAC present is 1:0.5. According to another embodiment, the molar ratio of the dose of acetaminophen to the therapeutic efficacy-enhancing amount of NAC present is 1:0.25. According to another embodiment, the molar ratio of the dose of acetaminophen to the therapeutic efficacy-enhancing amount of NAC present is 1:0.125. According to another embodiment, the molar ratio of the dose of acetaminophen to the therapeutic efficacy-enhancing amount of NAC present is 1:0.0625. According to another embodiment, the molar ratio of the dose of acetaminophen to the therapeutic efficacy-enhancing amount of NAC present is 1:0.03125. According to another embodiment, the molar ratio of the dose of acetaminophen to the therapeutic efficacy-enhancing amount of NAC present is 1:0.015625. According to another embodiment, the molar ratio of the dose of acetaminophen to the therapeutic efficacy-enhancing amount of NAC present is 1:00078125. According to another embodiment, the molar ratio of the dose of acetaminophen to the therapeutic efficacy-enhancing amount of NAC present is 1:0.00390625. According to another embodiment, the molar ratio of the dose of acetaminophen to the therapeutic efficacy-enhancing amount of NAC present is 1:0.001953125. According to another embodiment, the molar ratio of the dose of acetaminophen to the therapeutic efficacy-enhancing amount of NAC present is 1:0.000977.

Accordingly, the described invention allows for enhanced therapeutic efficacy of acetaminophen upon co-administration of NAC. Further, the therapeutic efficacy of the described acetaminophen-NAC compositions does not decrease during long term use of the acetaminophen-NAC composition. Without being limited by theory. NAC, which restores GSH, and can reduce symptoms, such as, but not limited to, fever and pain, supplements the antipyretic and analgesic activity of acetaminophen allowing for lower doses of acetaminophen to be used to achieve the same therapeutic effect as would be achieved with higher doses of unsupplemented acetaminophen. This increase in efficacy is proportional to the amount of NAC co-administered.

According to one embodiment of the method, the unit dose of acetaminophen in the composition is a dose that is less than a standard maximum daily dose of acetaminophen, wherein the standard maximum daily dose of acetaminophen is 200 mg/day, 400 mg/day, 600 mg/day, 800 mg/day, 1200 mg/day, 1600 mg/day, 1625 mg/day, 2000 mg/day, 2400 mg/day, 3200 mg/day, or 4000 mg/day, and the composition has a therapeutic effectiveness equivalent to the therapeutic effectiveness of acetaminophen alone when acetaminophen is administered at the standard maximum daily dose of acetaminophen.

General principles for determining therapeutic effectiveness, which may be found in Chapter 1 of Goodman and Gilman's The Pharmacological Basis of Therapeutics, 10th Edition, McGraw-Hill (New York) (2001) are summarized below.

Pharmacokinetic principles provide a basis for modifying a dosage regimen to obtain a desired degree of therapeutic efficacy with a minimum of unacceptable adverse effects. In situations where the drug's plasma concentration can be measured and related to the therapeutic window, additional guidance for dosage modification can be obtained.

Drug products are considered to be pharmaceutical equivalents if they contain the same active ingredients and are identical in strength or concentration, dosage form, and route of administration. Two pharmaceutically equivalent drug products are considered to be bioequivalent when the rates and extents of bioavailability of the active ingredient in the two products are not significantly different under suitable test conditions.

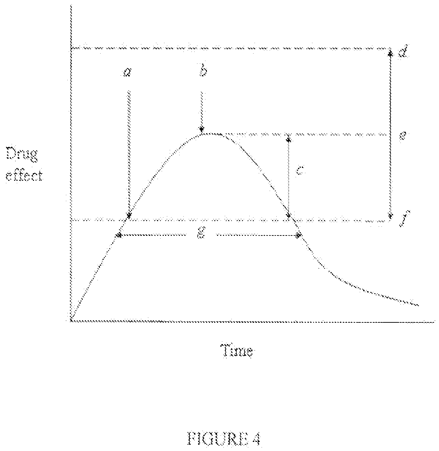

The term "therapeutic window" refers to a concentration range that provides therapeutic efficacy without unacceptable toxicity. Following administration of a dose of a drug, its effects usually show a characteristic temporal pattern, which is depicted in Figure 4. A lag period is present before the drug concentration exceeds the minimum effective concentration ("MEC") for the desired effect. Following onset of the response, the intensity of the effect increases as the drug continues to be absorbed and distributed. This reaches a peak, after which drug elimination results in a decline in the effect's intensity that disappears when the drug concentration falls back below the MEC. Accordingly, the duration of a drug's action is determined by the time period over which concentrations exceed the MEC. The therapeutic goal is to obtain and maintain concentrations within the therapeutic window for the desired response with a minimum of toxicity. Drug response below the MEC for the desired effect will be subtherapeutic, whereas for an adverse effect, the probability of toxicity will increase above the MEC. Increasing or decreasing drug dosage shifts the response curve up.or down the intensity scale and is used to modulate the drug's effect. Increasing the dose also prolongs a drug's duration of action but at the risk of increasing the likelihood of adverse effects. Accordingly, unless the drug is nontoxic, increasing the dose is not a useful strategy for extending a drug's duration of action. Instead, another dose of drug should be given to maintain concentrations within the therapeutic window. In general, the lower limit of the therapeutic range of a drug appears to be approximately equal to the drug concentration that produces about half of the greatest possible therapeutic effect, and the upper limit of the therapeutic range is such that no more than about 5% to about 10% of patients will experience a toxic effect. Of course, these figures can be highly variable, and some patients may benefit greatly from drug concentrations that exceed the therapeutic range, while others may suffer significant toxicity at much lower values. The therapeutic goal is to maintain steady-state drug levels within the therapeutic window. For most drugs, the actual concentrations associated with this desired range are not and need not be known, and it is sufficient to understand that efficacy and toxicity are generally concentration-dependent, and how drug dosage and frequency of administration affect the drug level. For a small number of drugs where there is a small (two- to three-fold) difference between concentrations resulting in efficacy and toxicity, a plasma-concentration range associated with effective therapy has been defined. In this case, a target level strategy is reasonable, wherein a desired target steady-state concentration of the drug (usually in plasma) associated with efficacy and minimal toxicity is chosen, and a dosage is computed that is expected to achieve this value. Drug concentrations subsequently are measured and dosage is adjusted if necessary to approximate the target more closely.

In most clinical situations, drugs are administered in a series of repetitive doses or as a continuous infusion to maintain a steady-state concentration of drug associated with the therapeutic window. To maintain the chosen steady-state or target concentration ("maintenance dose"), the rate of drug administration is adjusted such that the rate of input equals the rate of loss. If the clinician chooses the desired concentration of drug in plasma and knows the clearance and bioavailability for that drug in a particular patient, the appropriate dose and dosing interval can be calculated.

The "loading dose" is one or a series of doses that may be given at the onset of therapy with the aim of achieving the target concentration rapidly. A loading dose may be desirable if the time required to attain steady state by the administration of drug at a constant rate is long relative to the temporal demands of the condition being treated.

The precise dose of APAP and NAC to be employed in an APAP-NAC formulation will depend on the route of administration and the seriousness of the disease or disorder, and should be decided according to the judgment of the practitioner and each patient's circumstances. See, for example, Goodman and Gilman's The Pharmacological Basis of Therapeutics (2001); The Physician's Desk Reference, Medical Economics Company, Inc., Oradell, N.J., 1995; and to Drug Facts and Comparisons, Facts and Comparisons, Inc., St. Louis, Mo., 1993. Patients on therapy known to deplete cysteine/glutathione or produce oxidative stress may benefit from higher amounts of NAC One of ordinary skill in the art may determine empirically the effective amount of a composition comprising acetaminophen and NAC without necessitating undue experimentation. Generally, it is preferred that a maximum dose be used, that is, the highest safe dose according to some medical judgment. Accordingly, for any composition described herein, the therapeutically effective amount may be initially determined from preliminary *in vitro* studies and/or animal models. A therapeutically effective dose also may be determined from human data. The applied dose may be adjusted based on the relative bioavailability and potency of the administered compound. Adjusting the dose to achieve maximal efficacy based on the methods described above and other methods are well-known in the art and is well within the capabilities of the ordinarily skilled artisan.

The terms "minimize", "reduce" and their grammatical equivalents refer to a reduction in the frequency arid or severity of one or more adverse drug experiences in a given subject or subject population. It is appreciated that the subject population may be of necessity much smaller in size than the general population that may be exposed to the drug and/or its adverse experiences.

Routine serum liver chemistries (bilirubin, aspartate aminotransferase (AST), Alanine aminotransferase (ALT), alkaline phosphatase) and serum alpha glutathione S-transferase (αGST), may be used as a measure of hepatocellular injury. It also is appreciated that the results obtained from methods for determining the reduction in the frequency and/or severity of adverse drug experiences may be subject to variables such as intra-subject and inter-subject factors. However, it also is appreciated that certain scientifically accepted methods can be used to conduct the studies and that the results from such studies are statistically reliable. Such methods and interpretation of the results from such methods are well-known in the art. See, for example, Robert R. Sokal & F. James Rohlf, Biometry: The Principles and Practice of Statistics in Biological Research, 2nd ed. (1969), which is incorporated by reference in its entirety.

### NAC Packaging: Stability

Over-the-counter NAC can be produced variably and packaged. Because production and packaging methods generally do not guard against oxidation, NAC can be significantly contaminated with bioactive oxidation products. These may be particularly important in view of data indicating that the oxidized form of NAC has effects counter to those reported for NAC and is bioactive at doses roughly 10-100 fold less than NAC (see Samstrand et al (1999) J. Pharmacol. Exp. Ther. 288: 1174-84).

The distribution of the oxidation states of NAC as a thiol and disulfide depends on the oxidation/reduction potential. The half-cell potential obtained for the NAC thiol/disulfide pair is about +63 mV, indicative of its strong reducing activity among natural compounds (see Noszal et al. (2000) J. Med. Chem. 43:2176-2182).

It therefore is highly desirable that the APAP-NAC formulation is prepared and stored so that oxidation of the reduced form of NAC is minimized. When in solution, NAC containing formulations may be stored in a brown bottle that is vacuum sealed. In some embodiments, storage is in a cool dark environment. In some embodiments, NAC containing formulations in solid form are blister packed under gas. In some embodiments, APAP:NAC formulations are formulated as a tablet, wherein the tablet comprises antioxidants. In some such embodiments, the tablet is uncoated. In some such embodiments, the tablet is coated with a coating that acts to, for example, limit oxygen transfer or photolability. According to another embodiment, the composition further comprises stabilizing agents. Stabilizing agents may include, but are not limited to, antioxidants. Such agents may act to, for example, but not limited to, inhibit oxygen transfer or photolability.

The determination of reduced and oxidized species present in a sample may be determined by various methods known in the art, for example, with capillary electrophoresis, HPLC, etc. as described by Chassaing ct al. (1999) J Chromatogr B Biomed Sci Appl 735(2):219-27.

### Administration

The formulations of the described invention may be administered orally, topically, parenterally, buccally, by inhalation or insufflation (either through the.mouth or through the nose), rectally, or by any means known to the skilled artisan. The composition can be a liquid solution, suspension, emulsion, tablet, pill, capsule, sustained release formulation, powder, or can be formulated as a suppository, with traditional binders and carriers such as triglycerides.

### Oral Delivery

The compositions of the described invention may be in a form suitable for oral use, for example, as tablets, troches, lozenges, aqueous or oily suspensions, dispersible powders or granules, emulsions, hard or soft capsules or syrups or elixirs. As used herein, the terms "oral" or "orally" refer to the introduction into the body by mouth whereby absorption occurs in one or more or the following areas of the body: the mouth, stomach, small intestine, lungs (also specifically referred to as inhalation), and the small blood vessels under the tongue (also specifically referred to as sublingually). Compositions intended for oral use may be prepared according to any known method, and such compositions may contain one or more agents selected from the group consisting of sweetening agents, flavoring agents, coloring agents, and preserving agents in order to provide pharmaceutically elegant and palatable preparations. Tablets may contain the active ingredient(s) in admixture with non-toxic pharmaceutically-acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, com starch or alginic acid; binding agents, for example, starch, gelatin or acacia; and lubricating agents, for example, magnesium stearate, stearic acid or talc. The tablets may be uncoated or they may be coated by known techniques, for example, to delay disintegration and absorption in the gastrointestinal tract and thereby provide a sustained action over a longer period, to protect the composition from oxidation or photodegradation; or for controlled release. For example, a time delay material such as glyceryl monostearate or glyceryl distearate may be employed.

Compositions of the described invention also may be formulated for oral use as hard gelatin capsules, where the active ingredient(s) is(are) mixed with an inert solid diluent, for example, calcium carbonate, calcium phosphate or kaolin, or soft gelatin capsules wherein the active ingredient(s) is (are) mixed with water or an oil medium, for example, peanut oil, liquid paraffin, or olive oil.

The compositions of the described invention may be formulated as aqueous suspensions wherein the active ingredient(s) is (are) in admixture with excipients suitable for the manufacture of aqueous suspensions. Such excipients arc suspending agents, for example, sodium carboxymethylcellulose, methylcellulose, hydroxypropylmethylcellulose, sodium alginate, polyvinylpyrrolidone, gum tragacanth, and gum acacia; dispersing or wetting agents may be a naturally-occurring phosphatide such as lecithin, or condensation products of an alkylene oxide with fatty acids, for example, polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions also may contain one or more coloring agents, one or more flavoring agents, and one or more sweetening agents, such as sucrose or saccharin.

Compositions of the described invention may be formulated as oily suspensions by suspending the active ingredient in a vegetable oil, for example arachis oil, olive oil, sesame oil or coconut oil, or in a mineral oil, such as liquid paraffin. The oily suspensions may contain a thickening agent, for example, beeswax, hard paraffin or cetyl alcohol. Sweetening agents, such as those set forth above, and flavoring agents may be added to provide a palatable.oral preparation. These compositions may be preserved by the addition of an antioxidant such as ascorbic acid.

Compositions of the described invention may be formulated in the form of dispersible powders and granules suitable for preparation of an aqueous suspension by the addition of water. The active ingredient in such powders and granules is provided in admixture with a dispersing or wetting agent, suspending agent, and one or more preservatives. Suitable dispersing or wetting,agents and suspending agents are exemplified by those already mentioned above. Additional excipients, or example, sweetening, flavoring and coloring agents also may be present.

The compositions of the invention also may be in the form of an emulsion. An emulsion is a two-phase system prepared by combining two immiscible liquid carriers, one of which is disbursed uniformly throughout the other and consists of globules that have diameters equal to or greater than those of the largest colloidal particles. The globule size is critical and must be such that the system achieves maximum stability. Usually, separation of the two phases will not occur unless a third substance, an emulsifying agent, is incorporated. Thus, a basic emulsion contains at least three components, the two immiscible liquid carriers and the emulsifying agent, as well as the active ingredient. Most emulsions incorporate an aqueous phase into a non-aqueous phase (or vice versa). However, it is possible to prepare emulsions that arc basically non-aqueous, for example, anionic and cationic surfactants of the non-aqueous immiscible system glycerin and olive oil. Thus, the compositions of the invention may be in the form of an oil-in-water emulsion. The oily phase may be a vegetable oil, for example, olive oil or arachis oil, or a mineral oil, for example a liquid paraffin, or a mixture thereof. Suitable emulsifying agents may be naturally-occurring gums, for example, gum acacia or gum tragacanth, naturally-occurring phosphatidcs, for example soy bean, lecithin, and esters or partial esters derived from fatty acids and hexitol anhydrides, for example sorbitan monooleate, and condensation products of the partial esters with ethylene oxide, for example, polyoxyethylene sorbitan monooleate. The emulsions also may contain sweetening and flavoring agents.

The compositions of the invention also may be formulated as syrups and elixirs. Syrups and elixirs may be formulated with sweetening agents, for example, glycerol, propylene glycol, sorbitol or sucrose. Such formulations also may contain a demulcent, a preservative, and flavoring and coloring agents. Demulcents are protective agents employed primarily to alleviate irritation, particularly mucous membranes or abraded tissues. A number of chemical substances possess demulcent properties. These substances include the alginates, mucilages, gums, dextrins, starches, certain sugars, and polymeric polyhydric glycols. Others include acacia, agar, benzoin, carbomer, gelatin, glycerin, hydroxyethyl cellulose, hydroxypropyl cellulose, hydroxypropyl methylcellulose, propylene glycol, sodium alginate, tragacanth, hydrogels and the like.

For buccal administration, the compositions of the described invention may take the form of tablets or lozenges formulated in a conventional manner.

There are three general methods of tablet preparation: the wet-granulation method; the dry-granulation method; and direct compression. The method of preparation and the added ingredients are selected to give the tablet formulation the desirable physical characteristics allowing the rapid compression of tablets. After compression, the tablets must have a number of additional attributes such as appearance, hardness, disintegration ability, appropriate dissolution characteristics, and uniformity, which also are influenced both by the method of preparation and by the added materials present in the formulation.

According to another embodiment, the tablet is a compressed tablet (CT). Compressed tablets are solid dosage forms formed with pressure and contain no special coating. Generally, they are made from powdered, crystalline, or granular materials, alone or in combination with binders, disintegrants, controlled-release polymers, lubricants, diluents and colorants.

According to another embodiment, the tablet is a sugar-coated tablet. These are compressed tablets containing a sugar coating. Such coatings may be colored and are beneficial in covering up drug substances, possessing objectionable tastes or odors and in protecting materials sensitive to oxidation.

According to another embodiment, the tablet is a film-coated tablet. These Compressed tablets are covered with a thin layer or film of a water-soluble material. Numerous polymeric substances with film-forming properties may be used.

According to another embodiment, the tablet is an enteric-coated tablet. These Compressed tablets are coated with substances that resist solution in gastric fluid but disintegrate in the intestine.

According to another embodiment, the tablet is a multiple compressed tablet. These tablets are made by more than one compression cycle. Layered tablets are prepared by compressing additional tablet granulation on a previously compressed granulation. The operation may be repeated to produce multilayered tablets of two or three layers. Press-coated tablets (dry-coated) arc prepared by feeding previously compressed tablets into a special tableting machine and compressing another granulation layer around the preformed tablets.

According to another embodiment, the tablet is a controlled-release tablet. Compressed tablets can be formulated to release the drug slowly over a prolonged period of time. Hence, these dosage forms have been referred to as prolonged-release or sustained-release dosage forms.

According to another embodiment, the tablet is a tablet for solution. These Compressed tablets may be used to prepare solutions or to impart given characteristics to solutions.

According to some such embodiments, the tablet is an effervescent tablet. In addition to the drug, these tablets contain sodium bicarbonate and an organic acid such as tartaric acid or citric acid. In the presence of water, these additives react, liberating carbon dioxide that acts as a disintegrator and produce effervescence.

According to another embodiment, the tablet is a compressed suppository or insert.

According to another embodiment, the tablet is a buccal and or sublingual tablet. These are small, flat, oval tablets intended for buccal administration and that by inserting into the buccal pouch may dissolve or erode slowly.

According to another embodiment, the tablet is a molded tablet or tablet triturate.

In some embodiments, the tablet comprises a compressed core comprising at least one component of the described formulation; and a membrane forming composition. Formulations utilizing membrane forming compositions are known to those of skill in the art (see, for example, Remington's Pharmaceutical Sciences, 20th Ed., 2000). Such membrane forming compositions,may include, for example, a polymer, such as, but not limited to, cellulose ester, cellulose ether, and cellulose ester-ether polymers, an amphiphilic triblock copolymer surfactant, such as ethylene oxide-propylene oxide-ethylene oxide, and a solvent, such as acetone, which forms a membrane over the core. The compressed core may contain a bi-layer core including a drug layer and a push layer.

### Non-Oral Delivery

The term "non-oral administration" represents any method of administration in which a composition is not provided in a solid or liquid oral dosage form, wherein such solid or liquid oral dosage form is traditionally intended to substantially release and or deliver the drug in the gastrointestinal tract beyond the mouth and/or buccal cavity. Such solid dosage forms include conventional tablets, capsules, caplets, etc., which do not substantially release the drug in the mouth or in the oral cavity. It is appreciated that many oral liquid dosage forms such as solutions, suspensions, emulsions, etc., and some oral solid dosage forms may release some of the drug in the mouth or in the oral cavity during the swallowing of these formulations. However, due to their very short transit time through the mouth and the oral cavities, the release of drug from these formulations in the mouth or the oral cavity is considered de minimus or insubstantial.. Accordingly, it is understood that the term "non-oral" includes parenteral, transdermal, inhalation, implant, and vaginal or rectal formulations and administrations. Further, implant formulations arc to be included in the term "non-oral," regardless of the physical location of implantation. Particularly, implantation formulations arc known which are specifically designed for implantation and retention in the gastrointestinal tract. Such implants are also considered to be non-oral delivery formulations, and therefore, are encompassed by the term "non-oral."

### Rectal Delivery

The compositions of the described invention may be in the form of suppositories for rectal administration of the composition, such as for treating pediatric fever. "Rectal" or "rectally" as used herein refers to introduction into the body through the rectum where absorption occurs through the walls of the rectum. These compositions can be prepared by mixing the drug with a suitable nonirritating excipient such as cocoa butter and polyethylene glycols which arc solid at ordinary temperatures but liquid at the rectal temperature and will therefore melt in the rectum and release the drug. When formulated as a suppository the compositions of the invention may be formulated with traditional binders and carriers, such as triglycerides.

### Parenteral Delivery

The compositions of the described invention may be in the form of a sterile injectable aqueous or oleaginous suspension. The term "parenteral" as used herein refers to introduction into the body by way of an injection (i.e., administration by injection), including, for example, subcutaneously (i.e., an injection beneath the skin), intramuscularly (i.e., an injection into a muscle); intravenously (i.e., an injection into a vein), intrathecally (i.e., an injection into the space around the spinal cord), intrasternal injection, or infusion techniques. A parenterally administered composition of the described invention is delivered using a needle, e.g., a surgical needle. The term "surgical needle" as used herein, refers to any needle adapted for delivery of fluid (i.e., capable of flow) compositions of the described invention into a selected anatomical structure. Injectable preparations, such as sterile injectable aqueous or oleaginous suspensions, may be formulated according to the known art using suitable dispersing or wetting agents and suspending agents.

The sterile injectable preparation may also be a sterile injectable solution or suspension in a nontoxic parenterally acceptable diluent or solvent, for example, as a solution in 1, 3-butanediol. A solution generally is considered as a homogeneous mixture of two or more substances; it is frequently, though not necessarily, a liquid. In a solution, the molecules of the solute (or dissolved substance) are uniformly distributed among those of the solvent. A suspension is a dispersion (mixture) in which a finely-divided species is combined with another species, with the former being so finely divided and mixed that it doesn't rapidly settle out. In everyday life, the most common suspensions arc those of solids in liquid water. Among the acceptable vehicles and solvents that may be employed arc water, Ringer's solution, and isotonic sodium chloride solution. In addition, sterile, fixed oils are conventionally employed as a solvent or suspending medium. For parenteral application, particularly suitable vehicles consist of solutions, preferably oily or aqueous solutions, as well as suspensions, emulsions, or implants. Aqueous suspensions may contain substances which increase the viscosity of the suspension and include, for example, sodium carboxymethyl cellulose, sorbitol and/or dextran. Optionally, the suspension may also contain stabilizers.

### Delivery by Inhalation or Insufflation

The compositions of the described invention may be in the form of a dispersible dry powder for delivery by inhalation or insufflation (either through the mouth or through the nose). Dry powder compositions may be prepared by-processes known in the art, such as lyophilization and jet milling, as disclosed in International Patent Publication No. WO 91/16038 and as disclosed in U.S. Pat. No. 6,921,527. The composition of the described invention is placed within a suitable dosage receptacle in an amount sufficient to provide a subject with a unit dosage treatment. The dosage receptacle is one that fits within a suitable inhalation device to allow for the aerosolization of the dry powder composition by dispersion into a gas stream to form an aerosol and then capturing the aerosol so produced in a chamber having a mouthpiece attached for subsequent inhalation by a subject in need of treatment. Such a dosage receptacle includes any container enclosing the composition known in the art such as gelatin or plastic capsules with a removable portion that allows a stream of gas (e.g., air) to be directed into the container to disperse the dry powder composition. Such containers are exemplified by those shown in U.S. Pat. Nos. 4,227.522; U.S. Pat. No. 4,192,309; and U.S. Pat. No. 4,105,027. Suitable containers also include those used in conjunction with Glaxo's Ventolin® Rotohaler brand powder inhaler or Fison's Spinhaler® brand powder inhaler. Another suitable unit-dose container which provides a superior moisture barrier is formed from an aluminum foil plastic laminate. The pharmaceutical-based powder is filled by weight or by volume into the depression in the formable foil and hermetically scaled with a covering foil-plastic laminate. Such a container for use with a powder inhalation device is described in U.S. Pat. No. 4,778,054 and is used with Glaxo's Diskhaler® (U.S. Pat. Nos. 4.627,432; 4,811,731; and 5,035,237).

### Tropical Delivery

The term "topical" refers to administration of an inventive composition at, or immediately beneath, the point of application. The phrase "topically applying" describes application onto one or more surfaccs(s) including epithelial surfaces. Although topical administration, in contrast to transdermal administration, generally provides a local rather than a systemic effect, as used herein, unless otherwise stated or implied, the terms topical administration and transdermal administration are used interchangeably. For the purpose of this application, topical applications shall include mouthwashes and gargles.

Topical administration may also involve the use of transdermal administration such as transdermal patches or iontophoresis devices which are prepared according to techniques and procedures well known in the art. The terms "transdermal delivery system", transdermal patch" or "patch" refer to an adhesive system placed on the skin to deliver a time released dose of a drug(s) by passage from the dosage form through the skin to be available for distribution via the systemic circulation. Transdermal patches are a well-accepted technology used to deliver a wide variety of pharmaceuticals, including, but not limited to, scopolamine for motion sickness, nitroglycerin for treatment of angina pectoris, clonidine for hypertension, estradiol for post-menopausal indications, and nicotine for smoking cessation.

Patches suitable for use in the described invention include, but are not limited to, (1) the matrix patch; (2) the reservoir patch; (3) the multi-laminate drug-in-adhesive patch: and (4) the monolithic drug-in-adhesive patch; TRANSDERMAL AND TOPICAL DRUG DELIVERY SYSTEMS, pp. 249-297 (Tapash K. Ghosh et al, eds., 1997), hereby incorporated herein by reference. These patches are well known in the art and generally available commercially.

### Additional Components

The compositions of the described invention may further include conventional excipients, i.e., pharmaceutically acceptable organic or inorganic carrier substances suitable for parenteral application which do not deleteriously react with the active compounds. Suitable pharmaceutically acceptable carriers include, but arc not limited to, water, salt solutions, alcohol, vegetable oils, polyethylene glycols, gelatin, lactose, amylose, magnesium stearate, talc, silicic acid, viscous paraffin, perfume oil; fatty acid monoglycerides and diglycerides, petroethral fatty acid esters, hydroxymethylcellulose, polyvinylpyrrolidone, etc. The pharmaceutical preparations may be sterilized and if desired, mixed with auxiliary agents, e.g.. lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, suitable vehicles include solutions, such as oily or aqueous solutions, as well as suspensions, emulsions, or implants. Aqueous suspensions may contain substances which increase the viscosity of the suspension and include, for example, but not limited to, sodium carboxymethyl cellulose, sorbitol and/or dextran. Optionally, the suspension also may contain stabilizers.

The composition, if desired, also may contain minor amounts of wetting or emulsifying agents or pH buffering agents. Oral formulations can include standard carriers such as pharmaceutical grades of mannitol, lactose, starch, magnesium stearate, sodium saccharine, cellulose, magnesium carbonate, etc.

The term "pharmaceutically acceptable carrier" as used herein refers to any substantially non-toxic carrier conventionally useful for administration of pharmaceuticals in which the active component will remain stable and bioavailable. In some embodiments, the pharmaceutically acceptable carrier of the compositions of the described invention include a release agent such as a sustained release or delayed release carrier. In such embodiments, the carrier can be any material capable of sustained or delayed release of the active ingredient to provide a more efficient administration, resulting in less frequent and/or decreased dosage of the active ingredient, ease of handling, and extended or delayed effects. Non-limiting examples of such carriers include liposomes, microsponges, microspheres, or microcapsules of natural and synthetic polymers and the like. Liposomes may be formed from a variety of phospholipids such as cholesterol, stearylamines or phosphatidylcholines.

Additional compositions of the described invention can be readily prepared using technology which is known in the art such as described in Remington's Pharmaceutical Sciences, 18th or 19th editions, published by the Mack Publishing Company of Easton, Pennsylvania.

A therapeutically active agent can be formulated per se or in salt form. The term "pharmaceutically acceptable salt" means those salts which are, within the scope of sound medical judgment, suitable for use in contact with the tissues of humans and lower animals without undue toxicity, irritation, allergic response and the like and are commensurate with a reasonable benefit/risk ratio. Pharmaceutically acceptable salts arc well-known in the art. For example, P. H. Stahl, et al. describe pharmaceutically acceptable salts in detail in "Handbook of Pharmaceutical Salts: Properties, Selection, and Use" (Wiley VCH, Zurich, Switzerland: 2002). Pharmaceutically acceptable salts include, but are not limited to, those formed with free amino groups such as those derived from hydrochloric, phosphoric, sulfuric, acetic, oxalic, tartaric acids, etc., and those formed with free carboxyl groups such as those derived from sodium, potassium, ammonium, calcium, ferric hydroxides, isopropylamine, triethylaminc. 2-ethylamino ethanol, histidine, procaine, etc. When used in medicine the salts should be pharmaceutically acceptable, but non-pharmaceutically acceptable salts may conveniently be used to prepare pharmaceutically acceptable salts thereof. Other such salts include, but arc not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts may be prepared as alkaline metal or alkaline earth salts, such as sodium, potassium or calcium salts of the carboxylic acid group. The salts may be prepared *in situ* during the final isolation and purification of the compounds described within the present invention or separately by reacting a free base function with a suitable organic acid. Representative acid addition salts include, but are not limited to, acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate(isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups may be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid. Basic addition salts may be prepared *in situ* during the final isolation and purification of compounds described within the invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but arc not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammoniun, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like. Pharmaceutically acceptable salts may be also obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium or magnesium) salts of carboxylic acids may also be made.

The pharmaceutical agent or a pharmaceutically acceptable ester, salt, solvate or prodrug thereof may be mixed with additional active materials that do not impair the desired action, or with materials that supplement the desired action. Solutions or suspensions used for parenteral, or topical application may include, but are not limited to, for example, the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation may be enclosed in ampoules, disposable syringes or multiple dose vials made of glass or plastic. Administered intravenously, particular carriers are physiological saline or phosphate buffered saline (PBS).

### Packaging

From a manufacturing and quality control perspective, the compositions of the described invention can be produced and packaged in the same manner that NAC historically is manufactured. Because NAC destabilizes, degrades, and essentially weakens the longer it is exposed to air, it is manufactured employing strict temperature controls during the manufacturing process in order to limit exposure to air. In some embodiments, this process includes immediately packaging the tablet, capsule, or powder directly on the manufacturing line the moment each tablet is manufactured. Each tablet is individually wrapped and sealed in airtight packaging; optionally under gas, a process that has resulted in significant long term stability.

The described invention may be provided as a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. All technical and scientific terms used herein have the same meaning.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### Examples

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1. Veterinary applications of compositions comprising acetaminophen-N-acetylcysteine

Veterinarians have tried many preparations to provide analgesia to multiple animal species. Many of these species have greater sensitivity to the toxic effects of acetaminophen than humans. For example, toxicity in dogs is documented at doses > 40 mg/kg as compared to > 140 mg/kg in humans. The most extreme toxicity is in the cat family, where felines have demonstrated acetaminophen toxicity and death at doses as little as 5-10 mg/kg. This toxicity limits the therapeutic application of acetaminophen in animals.

According to the described invention, an animal in need of an antipyretic or analgesic is treated with a composition comprising a unit dose of APAP and a therapeutic efficacy- increasing amount of NAC such that acetaminophen and N-acetylcysteine arc present in the composition in a molar ratio of acetaminophen to N-acetyl cysteine ranging from about 1:15 to about 1:0.000977, without incurring the risk of toxicity due to oxidative stress or subject related decreases in cysteine/glutathione levels. The composition has a greater therapeutic effectiveness than the therapeutic effectiveness of acetaminophen alone when acetaminophen is administered at the unit dose of acetaminophen.

### Example 2. Chronic use of an acetaminophen-NAC composition by an adult patient to control osteoarthritis pain.

An adult patient takes a standard dose of 1000 mg acetaminophen four times a day for osteoarthritis pain. The patient receives the same pain relief by taking 750 mg of an acetaminophen-NAC four times a day, and the efficacy of the acetaminophen-NAC formulation docs not decrease during long term use of the acctaminophen-NAC composition.

### Example 3. Use of an acetaminophen-NAC composition to control fever refractory to acetaminophen in a pediatric patient.

A pediatric patient receives a standard maximum daily dose of acetaminophen to control a fever, but the acetaminophen is ineffective. The patient receives the same standard maximum daily dose of acetaminophen in the form of an acetaminophen-N-acetylcysteine composition. The fever is controlled at that dose.

While the invention has been described and illustrated with reference to certain preferred embodiments thereof, those skilled in the art will appreciate that various changes, modifications and substitutions can be made therein without departing from the scope of the invention as defined by the claims. For example, effective dosages other than the preferred dosages as set forth herein may be applicable as a consequence of variations in the responsiveness of the mammal being treated. Likewise, the specific pharmacological responses observed may vary according to and depending on the particular active compound selected or whether there are present pharmaceutical carriers, as well as the type of formulation and mode of administration employed, and such expected variations or differences in the results are contemplated in accordance with the objects and practices of the present invention.

### References:

1. Lee, C. S., Song, E. H., Park, S. Y., and Han, E. S. 2003. Combined effect of dopamine and MPP(+) on membrane permeability in mitochondria and cell viability in PC12 cells. Neurochem Int 43:147-54.
2. Ostapowicz, G., Fontana, R. J., Schiodt, F. V. Larson, A., Davern, T. J., Han, S. H., McCashland, T. M., Shakil, A. O., Hay, J. E., Hynan, L., Crippin, J. S.. Blei, A. T., Samuel, G., Reisch, J., and Lee. W. M. 2002. Results of a prospective study of acute liver failure at 17 tertiary care centers in the United States. Ann Intern Med 137:947-54*.*
3. Cctaruk, E. W., Dart. R. C.. Hurlbut, K. M. Horowitz, R. S., and Shih, R. 1997. Tylenol Extended Relief overdose. Ann Emerg Med 30:104-8.
4. Moling, O., Cairon, E., Rimenti, G., Pizza, F., Pristera, R., and Mian, P. 2006. Severe hepatotoxicity after therapeutic doses of acetaminophen. Clin Ther 28:755-60.
5. Linden. C.H. and Rumack, B. H. 1984. Acetaminophen overdose. Emerg Med Clin North Am 2:103-19.
6. Prescott, L. F. 2000. Paracetamol: past, present, and future. Am J Ther 7:143-7.
7. Prescott, L. F. and Critchley, J. A. 1983. The treatment of acetaminophen poisoning. Annu Rev Pharmacol Toxicol 23:87-101.

## Claims

1. A composition for use in a method of treating pain in a subject in need of analgesic relief, the composition comprising an oral formulation comprising:
(a) a unit dose of acetaminophen and
(b) a therapeutic efficacy-enhancing amount of N-acetylcysteine;
wherein a total of all unit doses to be administered per day according to said method is an amount less than a standard maximum daily dose of acetaminophen, wherein the standard maximum daily dose of acetaminophen is:
200 mg for a subject 0-3 months of age, 2.72-4.99 kg (6-11 lb) body weight, receiving 40 mg/dose;
400 mg for a subject 4-11 months of age, 5.44-7.71 kg (12-17 lb) body weight, receiving 80 mg/dose;
600 mg for a subject 12-23 months of age, 8.16-10.43 kg (18-23 lb) body weight, receiving 120 mg/dose;
800 mg for a subject 2-3 years of age, 10.89-15.85 kg (24-35 lb) body weight, receiving 160 mg/dose;
1200 mg for a subject 4-5 years of age, 16.33-21.32 kg (36-47 lb) body weight, receiving 240 mg/dose;
1600 mg for a subject 6-8 years of age, 21.77-26.76 kg (48-59 lb) body weight, receiving 320 mg/dose;
1625 mg for a subject 6-11 years of age, receiving 325 mg/dose;
2000 mg for a subject 9-10 years of age, 27.22-32.21 kg (60-71 lb) body weight, receiving 400 mg/dose;
2400 mg for a subject 11 years of age, 32.66-43.09 kg (72-95 lb) body weight, receiving 480 mg/dose;
3200 mg for a subject 12 years of age, 43.55 kg (96 lb) body weight receiving 640 mg/dose;
4000 mg for a subject >12 years of age to adult, receiving 650 mg/dose;
4000 mg for a subject >12 years of age to adult, receiving 500 mg/dose;
wherein
(i) acetaminophen and N-acetylcysteine are present in the composition in a molar ratio of acetaminophen to N-acetylcysteine ranging from 1:15 to 1:0.000977; and
(ii) the formulation is effective to provide pain relief equivalent to a standard maximum daily dose of acetaminophen.

2. A composition for use in a method of treating fever in a subject in need of antipyretic relief, the composition comprising an oral formulation comprising:
(a) a unit dose of acetaminophen and
(b) a therapeutic efficacy-enhancing amount of N-acetylcysteine;
wherein a total of all unit doses to be administered per day according to said method is an amount less than a standard maximum daily dose of acetaminophen, wherein the standard maximum daily dose of acetaminophen is:
200 mg for a subject 0-3 months of age, 2.72-4.99 kg (6-11 lb) body weight, receiving 40 mg/dose;
400 mg for a subject 4-11 months of age, 5.44-7.71 kg (12-17 lb) body weight, receiving 80 mg/dose;
600 mg for a subject 12-23 months of age, 8.16-10.43 kg (18-23 lb) body weight, receiving 120 mg/dose;
800 mg for a subject 2-3 years of age, 10.89-15.85 kg (24-35 lb) body weight, receiving 160 mg/dose;
1200 mg for a subject 4-5 years of age, 16.33-21.32 kg (36-47 lb) body weight, receiving 240 mg/dose;
1600 mg for a subject 6-8 years of age, 21.77-26.76 kg (48-59 lb) body weight, receiving 320 mg/dose;
1625 mg for a subject 6-11 years of age, receiving 325 mg/dose;
2000 mg for a subject 9-10 years of age, 27.22-32.21 kg (60-71 lb) body weight, receiving 400 mg/dose;
2400 mg for a subject 11 years of age, 32.66-43.09 kg (72-95 lb) body weight, receiving 480 mg/dose;
3200 mg for a subject 12 years of age, 43.55 kg (96 lb) body weight receiving 640 mg/dose;
4000 mg for a subject >12 years of age to adult, receiving 650 mg/dose;
4000 mg for a subject >12 years of age to adult, receiving 500 mg/dose;
wherein
(i) acetaminophen and N-acetylcysteine are present in the composition in a molar ratio of acetaminophen to N-acetylcysteine ranging from 1:15 to 1:0.000977; and
(ii) the formulation is effective to provide antipyretic relief equivalent to a standard maximum daily dose of acetaminophen.

3. The composition for use according to claim 1 or claim 2, wherein the composition is an oral formulation selected from a liquid solution, a syrup, an elixir, a suspension, an emulsion, a tablet, a capsule, a sustained release formulation, or a powder.

4. The composition for use according to claim 3, wherein the tablet is a compressed tablet.

5. The composition for use according to claim 3, wherein the tablet is a coated tablet.

6. The composition for use according to claim 3, wherein the tablet is an effervescent tablet.

7. A composition for use in a therapeutic method for improving therapeutic efficacy of acetaminophen at a less than standard maximum daily dose, the composition comprising an oral formulation comprising:
(a) a unit dose of acetaminophen and
(b) a therapeutic efficacy-enhancing amount of N-acetylcysteine;
wherein a total of all unit doses to be administered per day is an amount less than a standard maximum daily dose of acetaminophen, wherein the standard maximum daily dose of acetaminophen is:
200 mg for a subject 0-3 months of age, 2.72-4.99 kg (6-11 lb) body weight, receiving 40 mg/dose;
400 mg for a subject 4-11 months of age, 5.44-7.71 kg (12-17 lb) body weight, receiving 80 mg/dose;
600 mg for a subject 12-23 months of age, 8.16-1.0.43 kg (18-23 lb) body weight, receiving 120 mg/dose;
800 mg for a subject 2-3 years of age, 10.89-15.85 kg (24-35 lb) body weight, receiving 160 mg/dose;
1200 mg for a subject 4-5 years of age, 16.33-21.32 kg (36-47 lb) body weight, receiving 240 mg/dose;
1600 mg for a subject 6-8 years of age, 21.77-26.76 kg (48-59 lb) body weight, receiving 320 mg/dose;
1625 mg for a subject 6-11 years of age, receiving 325 mg/dose;
2000 mg for a subject 9-10 years of age, 27.22-32.21 kg (60-71 lb) body weight, receiving 400 mg/dose;
2400 mg for a subject 11 years of age, 32.66-43.09 kg (72-95 lb) body weight, receiving 480 mg/dose;
3200 mg for a subject 12 years of age, 43.55 kg (96 lb) body weight receiving 640 mg/dose;
4000 mg for a subject >12 years of age to adult, receiving 650 mg/dose;
4000 mg for a subject >12 years of age to adult, receiving 500 mg/dose;
wherein
(i) acetaminophen and N-acetylcysteine are present in the composition in a molar ratio of acetaminophen to N-acetylcysteine ranging from 1:15 to 1:0.000977; and
(ii) the formulation is effective to provide antipyretic and analgesic relief equivalent to a standard maximum daily dose of acetaminophen without incurring risk of toxicity due to oxidative stress or subject related decreases in cysteine/glutathione levels.

8. The composition for use according to claim 7, wherein the composition is an oral formulation selected from a liquid solution, a syrup, an elixir, a suspension, an emulsion, a tablet, a capsule, a sustained release formulation, or a powder.

9. The composition for use according to claim 8, wherein the tablet is a compressed tablet.

10. The composition for use according to claim 8, wherein the tablet is a coated tablet.

11. The composition for use according to claim 8, wherein the tablet is an effervescent tablet.

## Patentansprüche

1. Zusammensetzung zur Verwendung in einem Verfahren zur Behandlung von Schmerzen eines Patienten, der eine Schmerzlinderung benötigt, wobei die Zusammensetzung eine orale Formulierung enthält, die umfasst:
(a) eine Einheitsdosis von Acetaminophen und
(b) eine die therapeutische Wirksamkeit verbessernde Menge von N-Acetylcystein;
wobei eine Gesamtmenge aller pro Tag verabreichter Einheitsdosen gemäß dem Verfahren eine Menge ist, die geringer als eine Standard-Tageshöchstdosis von Acetaminophen ist, wobei die Standard-Tageshöchstdosis von Acetaminophen beträgt:
200 mg für einen Patienten im Alter von 0-3 Monaten, 2,72-4,99 kg (6-11 lb) Körpergewicht, der 40 mg/Dosis erhält;
400 mg für einen Patienten im Alter von 4-11 Monaten, 5,44-7,71 kg (12-17 lb) Körpergewicht, der 80 mg/Dosis erhält;
600 mg für einen Patienten im Alter von 12-23 Monaten, 8,16-10,43 kg (18-23 lb) Körpergewicht, der 120 mg/Dosis erhält;
800 mg für einen Patienten im Alter von 2-3 Jahren, 10,89-15,85 kg (24-35 lb) Körpergewicht, der 160 mg/Dosis erhält;
1200 mg für einen Patienten im Alter von 4-5 Jahren, 16,33-21,32 kg (36-47 lb) Körpergewicht, der 240 mg/Dosis erhält;
1600 mg für einen Patienten im Alter von 6-8 Jahren, 21,77-26,76 kg (48-59 lb) Körpergewicht, der 320 mg/Dosis erhält;
1625 mg für einen Patienten im Alter von 6-11 Jahren, der 325 mg/Dosis erhält;
2000 mg für einen Patienten im Alter von 9-10 Jahren, 27,22-32,21 kg (60-71 lb) Körpergewicht, der 400 mg/Dosis erhält;
2400 mg für einen Patienten im Alter von 11 Jahren, 32,66-43,09 kg (72-95 lb) Körpergewicht, der 480 mg/Dosis erhält;
3200 mg für einen Patienten im Alter von 12 Jahren, 43,55 kg (96 lb) Körpergewicht, der 640 mg/Dosis erhält;
4000 mg für einen Patienten im Alter von mehr als 12 Jahren oder Erwachsenen, der 650 mg/Dosis erhält;
4000 mg für einen Patienten im Alter von mehr als 12 Jahren oder Erwachsenen, der 500 mg/Dosis erhält;
wobei
(i) Acetaminophen und N-Acetylcystein in der Zusammensetzung in einem Molverhältnis von Acetaminophen zu N-Acetylcystein vorliegt, welches im Bereich von 1:15 bis 1:0,000977 liegt;
(ii) die Formulierung die Wirkung hat, eine Schmerzlinderung zu bewirken, die äquivalent zu einer Standard-Tageshöchstdosis von Acetaminophen ist.

2. Zusammensetzung zur Verwendung in einem Verfahren zur Behandlung von Fieber in einem Patienten, der eine Fiebersenkung benötigt, wobei die Zusammensetzung eine orale Formulierung enthält, die umfasst:
(a) eine Einheitsdosis von Acetaminophen und
(b) eine die therapeutische Wirksamheit verbessernde Menge von N-Acetylcystein;
wobei eine Gesamtmenge aller pro Tag verabreichter Einheitsdosen gemäß dem Verfahren eine Menge ist, die geringer als eine Standard-Tageshöchstdosis von Acetaminophen ist, wobei die Standard-Tageshöchstdosis von Acetaminophen beträgt:
200 mg für einen Patienten im Alter von 0-3 Monaten, 2,72-4,99 kg (6-11 lb) Körpergewicht, der 40 mg/Dosis erhält;
400 mg für einen Patienten im Alter von 4-11 Monaten, 5,44-7,71 kg (12-17 lb) Körpergewicht, der 80 mg/Dosis erhält;
600 mg für einen Patienten im Alter von 12-23 Monaten, 8,16-10,43 kg (18-23 lb) Körpergewicht, der 120 mg/Dosis erhält;
800 mg für einen Patienten im Alter von 2-3 Jahren, 10,89-15,85 kg (24-35 lb) Körpergewicht, der 160 mg/Dosis erhält;
1200 mg für einen Patienten im Alter von 4-5 Jahren, 16,33-21,32 kg (36-47 lb) Körpergewicht, der 240 mg/Dosis erhält;
1600 mg für einen Patienten im Alter von 6-8 Jahren, 21,77-26,76 kg (48-59 lb) Körpergewicht, der 320 mg/Dosis erhält;
1625 mg für einen Patienten im Alter von 6-11 Jahren, der 325 mg/ Dosis erhält;
2000 mg für einen Patienten im Alter von 9-10 Jahren, 27,22-32,21 kg (60-71 lb) Körpergewicht, der 400 mg/Dosis erhält;
2400 mg für einen Patienten im Alter von 11 Jahren, 32,66-43,09 kg (72-95 lb) Körpergewicht, der 480 mg/Dosis erhält;
3200 mg für einen Patienten im Alter von 12 Jahren, 43,55 kg (96 lb) Körpergewicht, der 640 mg/Dosis erhält;
4000 mg für einen Patienten im Alter von mehr als 12 Jahren oder Erwachsenen, der 650 mg/Dosis erhält;
4000 mg für einen Patienten im Alter von mehr als 12 Jahren oder Erwachsenen, der 500 mg/Dosis erhält;
wobei
(i) Acetaminophen und N-Acetylcystein in der Zusammensetzung in einem Molverhältnis von Acetaminophen zu N-Acetylcystein vorliegt, welches im Bereich von 1:15 bis 1:0,000977 liegt;
(ii) die Formulierung die Wirkung hat, eine Fiebersenkung zu bewirken, die äquivalent zu einer Standard-Tageshöchstdosis von Acetaminophen ist.

3. Zusammensetzung zur Verwendung nach Anspruch 1 oder 2, wobei die Zusammensetzung eine orale Formulierung ist, die ausgewählt ist aus einer flüssigen Lösung, einem Sirup, einem Elixier, einer Suspension, einer Emulsion, einer Tablette, einer Kapsel, einer Formulierung mit verzögerter Freisetzung oder einem Pulver.

4. Zusammensetzung zur Verwendung gemäß Anspruch 3, wobei die Tablette eine komprimierte Tablette ist.

5. Zusammensetzung zur Verwendung gemäß Anspruch 3, wobei die Tablette eine beschichtete Tablette ist.

6. Zusammensetzung zur Verwendung gemäß Anspruch 3, wobei die Tablette eine Brausetablette ist.

7. Zusammensetzung zur Verwendung in einem therapeutischen Verfahren zur Verbesserung der therapeutischen Wirksamkeit von Acetaminophen bei weniger als einer Standard-Tageshöchstdosis, wobei die Zusammensetzung eine orale Formulierung umfasst, die enthält:
(a) eine Einheitsdosis von Acetaminophen und
(b) eine die therapeutische Wirksamheit verbessernde Menge von N-Acetylcystein;
wobei eine Gesamtmenge sämtlicher pro Tag verabreichter Einheitsdosen eine Menge ausmacht, die geringer als eine Standard-Tageshöchstdosis von Acetaminophen ist, wobei die Standard-Tageshöchstdosis von Acetaminophen beträgt:
200 mg für einen Patienten im Alter von 0-3 Monaten, 2,72-4,99 kg (6-11 lb) Körpergewicht, der 40 mg/Dosis erhält;
400 mg für einen Patienten im Alter von 4-11 Monaten, 5,44-7,71 kg (12-17 lb) Körpergewicht, der 80 mg/Dosis erhält;
600 mg für einen Patienten im Alter von 12-23 Monaten, 8,16-10,43 kg (18-23 lb) Körpergewicht, der 120 mg/Dosis erhält;
800 mg für einen Patienten im Alter von 2-3 Jahren, 10,89-15,85 kg (24-35 lb) Körpergewicht, der 160 mg/Dosis erhält;
1200 mg für einen Patienten im Alter von 4-5 Jahren, 16,33-21,32 kg (36-47 lb) Körpergewicht, der 240 mg/Dosis erhält;
1600 mg für einen Patienten im Alter von 6-8 Jahren, 21,77-26,76 kg (48-59 lb) Körpergewicht, der 320 mg/Dosis erhält;
1625 mg für einen Patienten im Alter von 6-11 Jahren, der 325 mg/ Dosis erhält;
2000 mg für einen Patienten im Alter von 9-10 Jahren, 27,22-32,21 kg (60-71 lb) Körpergewicht, der 400 mg/Dosis erhält;
2400 mg für einen Patienten im Alter von 11 Jahren, 32,66-43,09 kg (72-95 lb) Körpergewicht, der 480 mg/Dosis erhält;
3200 mg für einen Patienten im Alter von 12 Jahren, 43,55 kg (96 lb) Körpergewicht, der 640 mg/Dosis erhält;
4000 mg für einen Patienten im Alter von mehr als 12 Jahren oder Erwachsenen, der 650 mg/Dosis erhält;
4000 mg für einen Patienten im Alter von mehr als 12 Jahren oder Erwachsenen, der 500 mg/Dosis erhält;
wobei
(i) Acetaminophen und N-Acetylcystein in der Zusammensetzung in einem Molverhältnis von Acetaminophen zu N-Acetylcystein vorliegt, welches im Bereich von 1:15 bis 1:0,000977 liegt;
(ii) die Formulierung so wirkt, dass sie eine Schmerzlinderung und Fiebersenkung bewirkt, die äquivalent zu derjenigen einer Standard-Tageshöchstdosis von Acetaminophen ist, ohne ein Risiko von Toxizität durch oxidativen Stress oder patientenbezogene Abnahmen im Cystein-/Glutathion-Gehalt zu riskieren.

8. Zusammensetzung zur Verwendung nach Anspruch 7, wobei die Zusammensetzung eine orale Formulierung ist, die ausgewählt ist aus einer flüssigen Lösung, einem Sirup, einem Elixier, einer Suspension, einer Emulsion, einer Tablette, einer Kapsel, einer Formulierung zur verzögerten Freisetzung oder einem Pulver.

9. Zusammensetzung zur Verwendung gemäß Anspruch 8, wobei die Tablette eine komprimierte Tablette ist.

10. Zusammensetzung zur Verwendung nach Anspruch 8, wobei die Tablette eine beschichtete Tablette ist.

11. Zusammensetzung zur Verwendung nach Anspruch 8, wobei die Tablette eine Brausetablette ist.

## Revendications

1. Une composition pour être utilisée dans un procédé de traitement de la douleur chez un patient nécessitant un soulagement par un analgésique, la composition comprenant une formulation pour un usage oral comprenant:
(a) une dose unitaire d'acétaminophène, et,
(b) une quantité de la N-acétylcystéine améliorant l'efficacité thérapeutique;
dans ladite composition la totalité de toutes les doses unitaires à administrer quotidiennement selon ledit procédé est une quantité inférieure à une dose quotidienne maximum standardisée d'acétaminophène, dans laquelle la dose quotidienne maximum standardisée d'acétaminophène est de:
- 200 mg pour un sujet âgé de 0-3 mois, ayant un poids corporel de 2,72-4,99 kg (6-11 lb) et prenant 40 mg/dose;
- 400 mg pour un sujet âgé de 4-11 mois, ayant un poids corporel de 5,44-7,71 kg (12-17 lb) et prenant 80 mg/dose;
- 600 mg pour un sujet âgé de 12-23 mois, ayant un poids corporel 8,16-10,43 kg (18-23 lb) et prenant 120 mg/dose;
- 800 mg pour un sujet âgé de 2-3 ans, ayant un poids corporelde 10,89-15,85 kg (24-35 lb) et prenant 160 mg/dose;
- 1200 mg pour un sujet âgé de 4-5 ans, ayant un poids corporel de 16,33-21,32 kg (36-47 lb) et prenant 240 mg/dose;
- 1600 mg pour un sujet âgé de 6-8 ans, ayant un poids corporel de 21,77-26,76 kg (48-59 lb) et prenant 320 mg/dose;
- 1625 mg pour un sujet âgé de 6-11 ans et prenant 325 mg/dose;
- 2000 mg pour un sujet âgé de 9-10 ans et de 27,22-32,21 kg (60-71 lb) de poids corporel et prenant 400 mg/dose;
- 2400 mg pour un sujet âgé de 11 ans, ayant un poids corporel 32,66-43,09 kg (72-95 lb) et prenant 480 mg/dose;
- 3200 mg pour un sujet âgé de 12 ans, ayant un poids corporel de 43,55 kg (96 lb) et prenant 640 mg/dose;
- 4000 mg pour un sujet dont l'âge est supérieur à 12 ans et jusqu'à l'âge adulte et prenant 650 mg/dose;
- 4000 mg pour un sujet dont l'âge est supérieur à 12 ans et jusqu'à l'âge adulte et prenant 500 mg/dose;
composition dans laquelle:
(i) l'acétaminophène et la N-acétylcystéine sont présents dans ladite composition selon un rapport molaire acétaminophène/N-acétylcystéine compris entre 1/15 et 1/0,000977,
(ii) la formulation est efficace pour fournir un soulagement de la douleur équivalent à une dose quotidienne maximum standardisée d'acétaminophène.

2. Une composition pour être utilisée dans un procédé de traitement de la fièvre chez un patient nécessitant un soulagement par un antipyrétique, la composition comprenant une formulation pour un usage oral comprenant
(a) une dose unitaire d'acétaminophène, et,
(b) une quantité de la N-acétylcystéine améliorant l'efficacité thérapeutique,
dans ladite composition la totalité de toutes les doses unitaires à administrer quotidiennement selon ledit procédé est une quantité inférieure à une dose quotidienne maximum standardisée d'acétaminophène, dans laquelle la dose quotidienne maximum standardisée d'acétaminophène est de:
- 200 mg pour un sujet âgé de 0-3 mois, ayant un poids corporel de 2.72-4.99 kg (6-11 lb) et prenant 40mg/dose;
- 400 mg pour un sujet âgé de 4-11 mois, ayant un poids corporel de 5,44-7,71 kg (12-17 lb) et prenant 80mg/dose;
- 600 mg pour un sujet âgé de 12-23 mois, ayant un poids corporel de 8,16-10,43 kg (18-23 lb) et prenant 120mg/dose;
- 800 mg pour un sujet âgé de 2-3 ans, ayant un poids corporel de 10,89-15,85 kg (24-35 lb) et prenant 160 mg/dose;
- 1200 mg pour un sujet âgé de 4-5 ans, ayant un poids corporel de 16,33-21,32 kg (36-47 lb) et prenant 240mg/dose;
- 1600 mg pour un sujet âgé de 6-8 ans, ayant un poids corporel de 21,77-26,76 kg (48-59 lb) et prenant 320mg/dose;
- 1625 mg pour un sujet âgé de 6-11 ans et prenant 325 mg/dose;
- 2000 mg pour un sujet âgé de 9-10 ans, ayant un poids corporel de 27,22-32.21 kg (60-71 lb) et prenant 400 mg/dose;
- 2400 mg pour un sujet âgé de 11 ans, ayant un poids corporel de 32.66-43,09 kg (72-95 lb) et prenant 480 mg/dose;
- 3200 mg pour un sujet âgé de 12 ans, ayant un poids corporel de 43,55 kg (96 lb) et prenant 640 mg/dose;
- 4000 mg pour un sujet dont l'âge est supérieur à 12 ans et jusqu'à l'âge adulte et prenant 650 mg/dose;
- 4000 mg pour un sujet dont l'âge est supérieur à 12 ans et jusqu'à l'âge adulte et prenant 500 mg/dose;
composition dans laquelle:
(i) l'acétaminophène et la N-acétylcystéine sont présents dans ladite composition selon un rapport molaire acétaminophène/N-acétylcystéine compris entre 1/15 et 1/0,000977, et
(ii) la formulation est efficace pour fournir un soulagement antipyrétique équivalent à une dose quotidienne maximum standardisée d'acétaminophène.

3. La composition pour être utilisée selon la revendication 1 ou 2, dans laquelle ladite composition est une formulation pour un usage oral choisie parmi les solutions liquide, sirop, élixir, suspension, émulsion, comprimé, capsule, formulation à libération progressive ou poudre.

4. La composition pour être utilisée selon la revendication 3, dans laquelle le comprimé est un comprimé obtenu par compression.

5. La composition pour être utilisée selon la revendication 3, dans laquelle le comprimé est un comprimé muni d'un revêtement.

6. La composition pour être utilisée selon la revendication 3, dans laquelle le comprimé est un comprimé effervescent.

7. Une composition pour être utilisée dans un procédé thérapeutique pour améliorer l'efficacité thérapeutique de l'acétaminophène en une quantité inférieure à une dose quotidienne maximum standardisée, la composition comprenant une formulation pour un usage oral comprenant:
(a) une dose unitaire d'acétaminophène, et,
(b) une quantité de la N-acétylcystéine améliorant l'efficacité thérapeutique,
dans ladite composition la totalité de toutes les doses unitaires à administrer quotidiennement selon ledit procédé est une quantité inférieure à une dose quotidienne maximum standardisée d'acétaminophène, dans laquelle la dose quotidienne maximum standardisée d'acétaminophène est de:
200 mg pour un sujet âgé de 0-3 mois, ayant un poids corporel de 2.72-4.99 kg (6-11 lb) et prenant 40 mg/dose;
- 400 mg pour un sujet âgé de 4-11 mois, ayant un poids corporel de 5,44-7,71 kg (12-17 1 b) et prenant 80 mg/dose ;
- 600 mg pour un sujet âgé de 12-23 mois, ayant un poids corporel de 8,16-10,43 kg (18-23 lb) et prenant 120mg/dose;
- 800 mg pour un sujet âgé de 2-3 ans, ayant un poids corporel de 10,89-15,85 kg (24-35 lb) et prenant 160mg/dose;
- 1200 mg pour un sujet âgé de 4-5 ans, ayant un poids corporel de 16,33-21,32 kg (36-47 lb) et prenant 240 mg/dose;
- 1600 mg pour un sujet âgé de 6-8 ans, ayant un poids corporel de 21,77-26,76 kg (48-59 lb) et prenant 320mg/dose;
- 1625 mg pour un sujet âgé de 6-11 ans et prenant 325 mg/dose;
- 2000 mg pour un sujet âgé de 9-10 ans, ayant un poids corporel de 27,22-32.21 kg (60-71 lb) et prenant 400 mg/dose;
- 2400 mg pour un sujet âgé de 11 ans, ayant un poids corporel de 32.66-43,09 kg (72-95 lb) et prenant 480 mg/dose;
- 3200 mg pour un sujet âgé de 12 ans, ayant un poids corporel de 43,55 kg (96 lb) et prenant 640 mg/dose;
- 4000 mg pour un sujet dont l'âge est supérieur à 12 ans et jusqu'à l'âge adulte et prenant 650 mg/dose;
- 4000 mg pour un sujet dont l'âge est supérieur à 12 ans et jusqu'à l'âge adulte et prenant 500 mg/dose;
composition dans laquelle:
(i) l'acétaminophène et la N-acétylcystéine sont présents dans ladite composition selon un rapport molaire acétaminophène/N-acétylcystéine compris entre 1/15 et 1/0,000977, et
(ii) la formulation est efficace pour fournir un soulagement antipyrétique et analgésique équivalent à une dose quotidienne maximum standardisée d'acétaminophène, sans provoquer une toxicité due au stress oxydatif ou à une diminution du taux de cystéine/glutathion en relation avec le sujet.

8. La composition pour être utilisée selon la revendication 7, dans laquelle ladite composition est une formulation pour un usage oral choisie parmi les solutions liquide, sirop, élixir, suspension, émulsion, comprimé, capsule, formulation à libération progressive ou poudre.

9. La composition pour être utilisée selon la revendication 8, dans laquelle le comprimé est un comprimé obtenu par compression

10. La composition pour être utilisée selon la revendication 8, dans laquelle le comprimé est un comprimé muni d'un revêtement.

11. La composition pour être utilisée selon la revendication 8, dans laquelle le comprimé est un comprimé effervescent.
